# EUROPEAN PATENT APPLICATION

(11) **EP 4 733 763 A1**
(43) Date of publication of application: **29.04.2026**
(21) Application number: 24208943.1
(22) Date of filing: 25.10.2024
(51) Int. Cl.: G01N 33/533, A61K 49/00, C07D 495/04, G01N 33/58

(54) **TRACER COMPOUNDS FOR WHOLE CELL LABELLING**

(71) Applicant: Charité - Universitätsmedizin Berlin Körperschaft des öffentlichen Rechts, 10117 Berlin (DE)
(72) Inventor: Turko, Paul, 13589 Berlin (DE); Vida, Imre, 13629 Berlin (DE); He, Zhengzheng, 10315 Berlin (DE); Barreda Tomás, Federico José, Tokyoto, 112-0015 (JP); Henklein, Petra, 12623 Berlin (DE)
(74) Representative: Hertin und Partner Rechts- und Patentanwälte PartG mbB

(57) **Abstract**

The invention relates to a tracer compound for whole-cell labelling, comprising: a non-streptavidin-binding biotin analogue, at least one connecting moiety comprising a functional group configured for a click-chemistry reaction, and at least one cross-linkable moiety, preferably wherein the cross-linkable moiety is a (para)formaldehyde cross-linkable group, and the non-streptavidin-binding biotin analogue enables the tracer compound to diffuse within a biological cell with essentially the same properties as biocytin and/or neurobiotin, preferably enabling the detection of cellular protrusions, such as dendrites, dendritic spines and axons. The invention further relates to methods for constructing such tracer compound and for labelling a cell therewith. The invention further relates to kits for carrying out the method according to the invention.

## Description

The invention is in the field of biology, specifically in the field of molecular biology and cell biology.

The invention relates to a tracer compound enabling the labelling or detection of electrically active cells, such as neurons, glia or muscle cells, by diffusing within the cell in a way that allows the detection of even intricate parts of the cell, such as cellular protrusions, and preferably without impairing the electrophysiological properties of the cell.

In particular the present invention relates to a tracer compound for whole-cell labelling, comprising: a non-streptavidin-binding biotin analogue, at least one connecting moiety comprising a functional group configured for a click-chemistry reaction, and at least one cross-linkable moiety, preferably wherein the cross-linkable moiety is a (para)formaldehyde cross-linkable group. The non-streptavidin-binding biotin analogue enables the tracer compound to diffuse within a biological cell with essentially the same properties as biocytin and/or neurobiotin, preferably enabling the detection of cellular protrusions, such as dendrites, dendritic spines and axons.

The invention further relates to methods for constructing such tracer compound and for labelling a cell therewith. The invention further relates to kits for carrying out the method according to the invention.

### BACKGROUND OF THE INVENTION

The development of novel tools and methodologies is essential for progress in neuroscience research. One of the most important ways of studying the electrical properties of neurons is through intracellular recordings, such as the whole-cell patch-clamp technique (Hamill et al., 1981). The technique involves recording a neuron's electrical activity from the inside, using a glass micropipette, filled with a suitable electrolyte solution and an electrode coupled to an amplifier. It is a widely used technique and is the gold standard for recording the synaptic inputs and intrinsic activity of neurons. The technique benefits from both a high sensitivity and temporal resolution and has been widely adopted, being used by thousands of research institutions across the globe. An additional advantage of the method is that it can be easily combined with the intracellular labelling of the recorded cell. This makes it possible to correlate a cell's electrical activity with its anatomical properties, allowing greater confidence in identification. Over the last decades, there have been significant advances in the whole-cell patch-clamp technique that have increased the number of cells that can be simultaneously recorded. Many laboratories previously only recorded from pairs of cells, however, more recently, it has become possible to record from 10 or even more cells simultaneously (Peng et al., 2017; Steiner et al., 2019; Peng et al., 2021). This has brought great advantages in terms of the number of cells and connections that can be tested simultaneously, but has raised major methodological challenges, in particular, for how to accurately identify the morphologies of large numbers of closely localized cells with overlapping neuronal processes.

Typically, following electrical recordings, cells are fixed, processed, and imaged, and their morphological features digitally reconstructed. For individual cells this process has become routine. However, as the number of cells increases, the process becomes extremely laborious or impossible, and can increase the chance for morphological errors. This is particularly problematic for strongly overlapping cells, such as neurons, which unlike other cells, in other organs, possess an extremely complicated morphology. This means that when 2 or more are in close proximity there can be a bewildering overlap of their fine dendritic and axonal processes. The time required for disentangling and correctly identifying overlapping processes is a major bottleneck in the analysis pipeline for multi-neuron identification and characterization.

At present, there is a critical shortage of suitable labelling molecules with which to label multiple neurons in discreet colors. Currently, the only effective molecules available for combined electrophysiology and imaging experiments are biocytin and its derivative neurobiotin. These molecules are widely considered to be the gold standard for neuronal labeling and the only ones that have achieved widespread application (Tasker et al., 1991; Thomson et al., 2011; Booker et al., 2014; Steiner et al., 2019). However, as the two molecules are visualized by the same avidin or streptavidin labelling molecule (conjugated to a fluorophore), their combined use, therefore does not permit multi-color labeling, limiting their labelling to only a single color. While other molecules exist, which permit some labelling of neuronal structures, problems with slow or incomplete labelling, blocking of the micropipette, altered electrophysiological properties, and complex processing steps have limited their application (Tasker et al., 1991; Takeuchi et al., 2008; Hanani et al., 2012; Ling et al., 2012: Parekh and Ascoli, 2013). Thus, at present, there are no equivalently applicable alternatives to biocytin (and neurobiotin) when it comes to high fidelity labelling coupled with a minimal influence on cellular physiology.

### SUMMARY OF THE INVENTION

In light of the prior art a technical problem underlying the present invention is to provide alternative or improved means for electrically neutral traces or dyes, which can be injected into cells during electrical recordings thereby labeling a cells entire structure without perturbing intracellular physiology.

Another aim of the present invention is the provision of molecules suitable for labelling cells that are compatible for combined use with biocytin and/or neurobiotin thereby enabling the distinguishable, selective visualization of multiple (interacting) cells.

This problem is solved by the features of the independent claims. Preferred embodiments of the present invention are provided by the dependent claims.

The invention therefore relates in a first aspect to a tracer compound for whole-cell labelling, comprising:
a. a non-streptavidin-binding biotin analogue,
b. at least one connecting moiety comprising at least one functional group configured for a click-chemistry reaction, and
c. at least one cross-linkable moiety.

In embodiments, the cross-linkable moiety is a (para)formaldehyde cross-linkable group, comprising an amine group, preferably an amino acid.

In embodiments, the non-streptavidin-binding biotin analogue enables the tracer compound to diffuse within a biological cell with essentially the same properties as biocytin and/or neurobiotin.

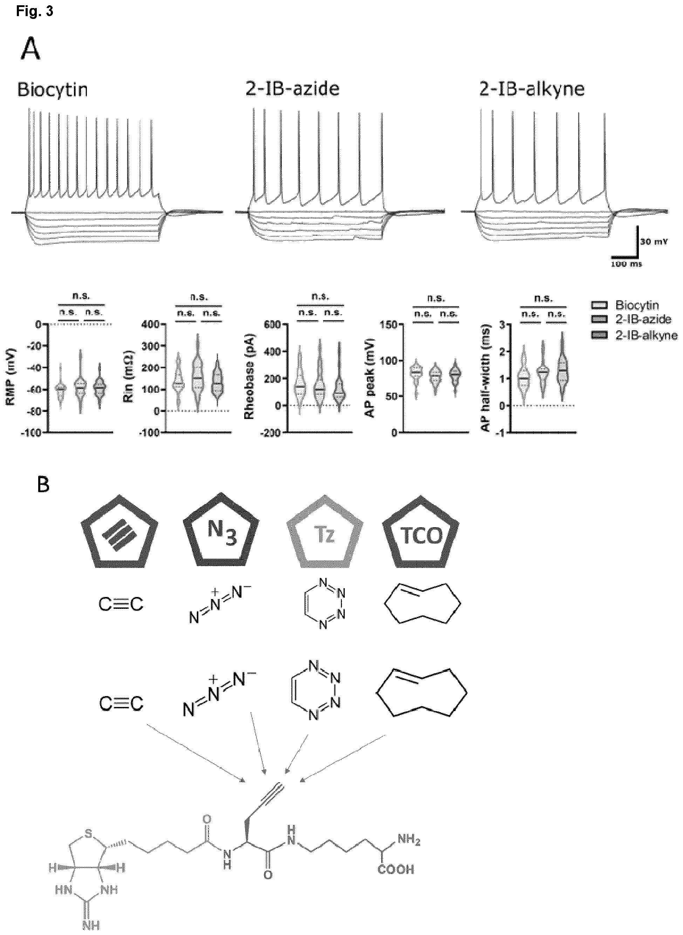

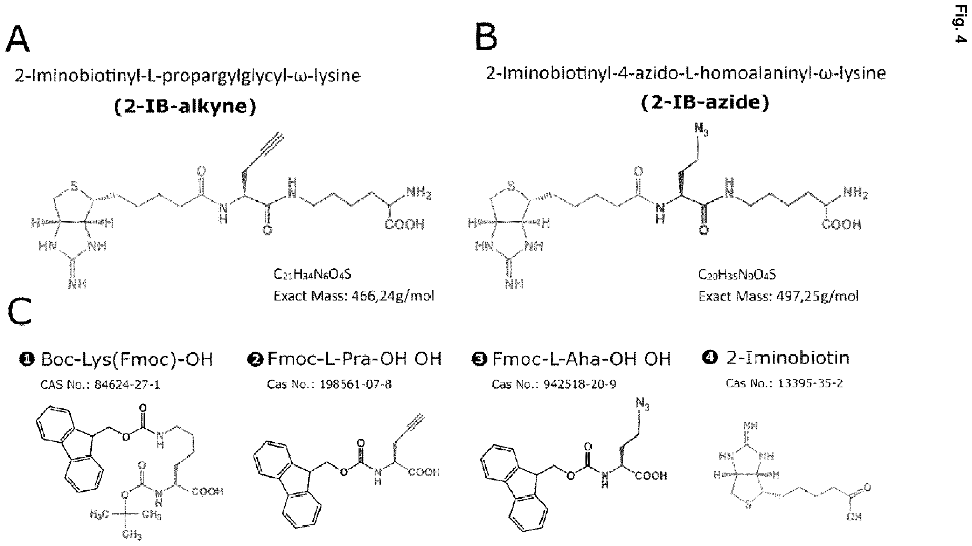

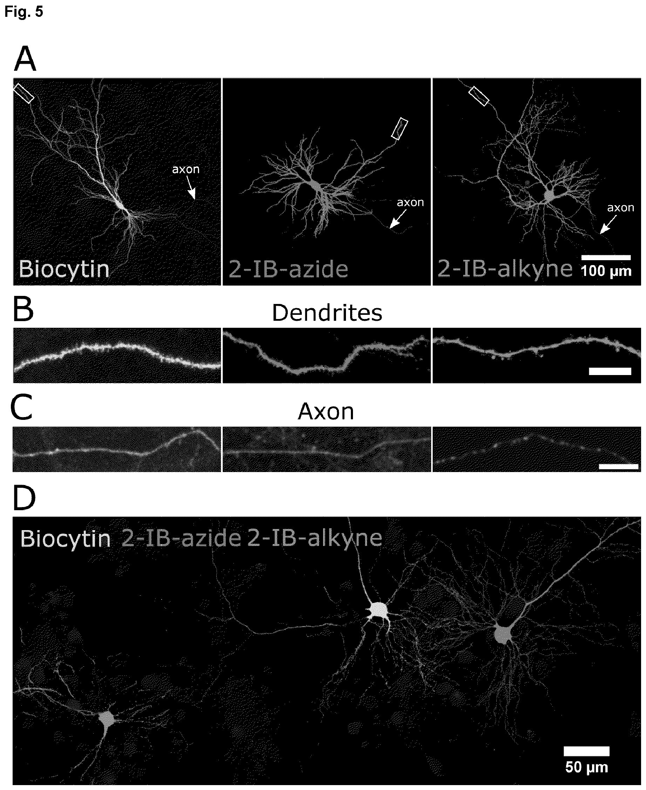

The present inventors aimed to address the above-mentioned shortcomings of the prior art by developing novel `electrically neutral' molecules, which can be injected into cells preferably during electrical recordings. An aim of the inventors was to develop molecules for visualizing multiple interacting cells, such as neurons or glia cells, thereby labeling a cell's entire structure, e.g., in case of neurons even fine processes, such as axons and dendritic spines. If electrically active cells, such as neurons or muscle cells are labelled, it can be of importance that the intracellular physiology of a cell is not affected or perturbed by the dye or tracer molecule. Additionally, the inventors aimed to engineer molecules which are compatible for combined use with biocytin. As can be seen in the following figures, the exemplary embodiments of the present invention, namely 2-IB-alkyne (2-Iminobiotinyl-L-propargylglycyl-w-lysine, Fig. 4A) and 2-IB-azide (2-lminobiotinyl-4-azido-L-homoalaninyl-w-lysine (Fig. 4B), can be used to intracellularly label the complex morphology of interacting cells (Fig. 5). Such molecules not only permit multi-color labelling of fine axonal and dendritic structures, but because they are also compatible with biocytin labelling and neuron physiology (Fig. 3), they therefore allow at least triple labelling of locally interacting cells. To the inventors knowledge, the molecules according to the present invention, such as e.g., 2-IB-alkyne and 2-IB-azide, are the first molecules to be newly designed in over 40 years which permit the detailed fluorescent labelling of fine neuronal structures without interfering with intrinsic electrical properties.

In embodiments, the cross-linkable moiety is a (para)formaldehyde cross-linkable group, comprising an amino acid, preferably lysine. In embodiments, the cross-linkable moiety is a (para)formaldehyde cross-linkable group comprising the amino acid lysine. In embodiments, the cross-linkable moiety is a (para)formaldehyde cross-linkable group, comprising an amino acid selected from lysine, arginine or histidine. In embodiments any amino acid comprising either a reactivity, and/or diffusability, etc. comparable to lysine, or comprising a different desired cross-linking pattern may be used within the cross-linkable group. A skilled person is familiar with means and methods for adapting reaction conditions or choosing amino acids depending on the desired cross-linking pattern.

In embodiments, an amino acid, such as lysine, is used because it is known to improve formaldehyde-mediated fixation of molecules to biological tissue (Tayri-Wilk et al., 2020). In other words, a cross-linkable moiety enables preferably the fixation of a tracer compound or dye within the cell by linking it to proteins within the cell.

In embodiments, a tracer compound molecule comprises three functional elements: as cross-linkable moiety a lysine residue, the biotin analogue molecule 2-iminobiotin (2-IB), and as connecting moiety at least one azide or alkyne functional group (see Fig. 4A for an exemplary, non-limiting embodiment).

In embodiments, the non-streptavidin-binding biotin analogue is a reversible-streptavidin biotin analogue.

In embodiments, the non-streptavidin-binding biotin analogue is 2-iminobiotin (2-IB). In embodiments, the non-streptavidin-binding or reversible-streptavidin biotin analogue is 2-iminobiotin (2-IB). The inventors selected as one embodiment 2-IB, because of its high structural similarity to biotin (the major component of biocytin; Athappilly et al., 1997). Another advantage of 2-IB is its greatly reduced binding affinity for streptavidin (Orr, 1981).

In embodiments, the non-streptavidin-binding biotin analogue (also) enables the tracer compound to diffuse within a biological cell, enabling the detection of cellular protrusions, such as dendrites, dendritic spines and axons, using microscopy, such as microscopic imaging.

It is in embodiments preferred that the present molecules comprise comparable or similar diffusion properties within a cell as biocytin or neurobiotin, such that the present compounds may be used in combination with said prior art dyes to stain, detect and analyze different cells, e.g., neighboring or interacting cells, in parallel and in a comparable manner. Particularly for cells comprising fine protrusions, e.g., such as axonal collaterals and dendritic spines, it can be advantageous, if a dye or tracer compound, such as biocytin or neurobiotin, is able to reach and diffuse into such intricate protrusions thereby enabling their detection.

The inventors postulated that the structural similarity of 2-iminobiotin (2-IB) to biotin would reduce the chance of unwanted toxic effects on tracer compound-filled cells, whereas the reduced binding affinity for streptavidin would make 2-IB compatible with labelling protocols for biocytin, without cross reaction. Surprisingly, the inventor's prototype testing confirmed these predictions and demonstrated excellent functionality, an absence of apparent toxic effects, and a lack of interference with biocytin labelling (Fig. 5 and 3A). Testing of the exemplary and non-limiting tracer compounds 2-IB-azide and 2-IB-alkyne revealed that each molecule can be used to label very fine structural components of neurons, including the axonal collaterals and dendritic spines (Fig. 5A-C). In addition, the preliminary testing showed that the exemplary tracer compounds 2-IB-azide and 2-IB-alkye can be used in addition/in parallel to biocytin to simultaneously `triple label' neighboring neurons (Fig. 5D). Finally, the inventors performed electrophysiological characterization of the exemplary prototype tracer compounds and found no evidence of toxicity or overt alterations to the intrinsic electrical properties of filled neurons (Fig 3A). The exemplary experimental data shown in the below examples therefore strongly support the thesis that the compounds according to the present invention are not only novel, but also non-toxic and biocytin-compatible labelling (tracer) molecules, which can in embodiments be used for combined morphological and electrophysiological investigations of cells with complex morphologies.

Hence, in embodiments, the tracer compound according to the present invention does not impair the electrophysiological properties of the cell (is electrophysiologically-neutral). In other words, the present tracer compounds preferably do not impair or affect electrophysiological measurements of a cell brought into contact with the present compound, such as during patch clamp measurements, or similar. The skilled person is familiar with common and suitable methods for measuring the electrophysiological properties of a cell.

In embodiments the present tracer compound does not show any toxic effects towards cells. In embodiments the present tracer compound does not interfere with the viability, the metabolism or other intracellular mechanisms of a cell and/or is free of (detectable or relevant) toxicity towards cells.

The present tracer compounds preferably do not (detectably) interfere with ion channel activity in a cell filled and/or stained therewith. The lack of interference with cellular ion channels is particularly relevant for electrophysiology measurements or methods. Hence, the present tracer compounds preferably do not (detectably) interfere with electrophysiology measurements of a cell (filled and/or stained therewith). In embodiments, the present tracer compounds have no discernible effects on the viability or other (intra)cellular mechanisms or properties. In embodiments, the present compounds do not show any of the above disadvantageous effects on a cell, preferably at least over the time span of at least an hour (after injection and/or upon contact), or even longer (preferably at least over a common duration of cellular electrophysiology measurements). In embodiments, the present compounds do not interfere with the viability, the metabolism and/or other intracellular mechanisms of a cell, and/or are toxic for the cell, preferably for at least 1 hour, or for at least 1-12 hours (after injection and/or upon contact). In preferred embodiments the tracer compounds are not (detectably) damaging or toxic (poisonous) to a cell filled and/or stained therewith.

Hence, in embodiments, the tracer compound according to the present invention preferably does not impair or affect the viability, the metabolism, the electrophysiological properties and/or other intracellular mechanisms of the cell.

In embodiments, the at least one functional group configured for a click-chemistry reaction is selected from the group comprising azides and alkynes, and preferably enables the generation of a triazole conjugation.

In embodiments, the at least one functional group configured for a click-chemistry reaction is selected from the group comprising azides, alkynes, tetra azides, trans-cycloctenes (TCO) and tetrazines.

In embodiments, the at least one functional group configured for a click-chemistry reaction is selected from the group comprising azides, alkynes, strained alkynes, alkenes, strained alkenes, tetra azides, cyclooctynes, trans-cycloctenes (TCO), phosphines, ketones, aldehydes, amines and tetrazines (Tz).

In embodiments, the at least one functional group configured for a click-chemistry reaction is selected from the group comprising tetra azides, trans-cycloctenes (TCO) and tetrazines.

In embodiments, the at least one functional group configured for a click-chemistry reaction is selected from the group comprising phosphines and azides. In embodiments, the at least one functional group configured for a click-chemistry reaction enables the generation of a Staudinger ligation, preferably between a phosphine and an azide.

In embodiments, the at least one functional group configured for a click-chemistry reaction is selected from the group comprising a strained alkyne, such as a cyclooctyne, and an azide. In embodiments, the at least one functional group configured for a click-chemistry reaction enables the generation of a triazole conjugation, preferably between a strained alkyne, such as a cyclooctyne, and an azide. In embodiments, the click-chemistry reaction between an azide and an (strained) alkyne is or comprises a Strain-Promoted Azide-Alkyne Cycloaddition (SPAAC) (a copper-free azide-alkyne cycloaddition), preferably wherein the strained alkyne, such as a cyclooctyne, reacts with an azide forming a triazole conjugation (preferably without the need for a copper catalyst).

In embodiments, the at least one functional group configured for a click-chemistry reaction is selected from the group comprising an electron-deficient diene, such as a tetrazine, and an electron-rich dienophile, such as a strained alkene or alkyne. In embodiments, the click-chemistry reaction is or comprises a Diels-Alder reaction (IEDDA), preferably wherein an electron-deficient diene, like tetrazine, reacts with an electron-rich dienophile, like a strained alkene or alkyne.

In embodiments, the click-chemistry reaction is an IEDDA reaction, preferably a tetrazine-trans-cyclooctene ligation (which is preferably biorthogonal).

In embodiments, the at least one functional group configured for a click-chemistry reaction is selected from the group comprising ketones or aldehydes, and amines. In embodiments, the at least one functional group configured for a click-chemistry reaction enables the generation of an oxime ligation, preferably between a ketone or aldehyde, and an amine.

In embodiments, the at least one functional group configured for a click-chemistry reaction is selected from the group comprising strained alkynes, such as dibenzoazacyclooctyne (DBCO), and azides. In embodiments, the at least one functional group configured for a click-chemistry reaction enables a strain-promoted azide-alkyne cycloaddition (SPAAC) reaction. In embodiments, the at least one functional group configured for a click-chemistry reaction enables a strain-promoted azide-alkyne cycloaddition reaction, preferably between a strained alkyne, such as dibenzoazacyclooctyne (DBCO), and an azide. This reaction preferably does not require copper, such as a `copper-free click-chemistry' reaction.

In embodiments a functional group can be conjugated to one or more reporter molecule(s), including, for example, fluorophores, fluorescent dyes or any functional equivalent thereof, via a click-chemistry reaction. In embodiments any of the functional groups of azides, alkynes, tetra azides, trans-cycloctene (TCO), tetrazines, strained alkynes, alkenes, strained alkenes, tetra azides, cyclooctynes, phosphines, ketones, aldehydes and amines can be conjugated to reporter molecules, including, for example, fluorophores (or any functional equivalent), via a click-chemistry reaction.

In embodiments, the click-chemistry reaction is or comprises a Diels-Alder reaction (IEDDA), a tetrazine-trans-cyclooctene ligation, a Staudinger ligation, a strain-promoted azide-alkyne cycloaddition (SPAAC) reaction, an oxime ligation or a copper(I)-catalysed azide-alkyne cycloaddition (CuAAC).

In embodiments any of the functional groups of azides, alkynes, tetra azides, trans-cycloctene (TCO) and tetrazinescan be conjugated to reporter molecules, including, for example, fluorophores, via Copper(I)-catalysed Azide-Alkyne Cycloaddition (CuAAC).

In embodiments fluorescent dyes, such as cyanine dyes, are functionalized with azide or alkyne group(s), enabling their conjugation to biomolecules via CuAAC.

In embodiments fluorescent dyes (e.g., cyanine dyes), fluorophores, or other dyes disclosed herein, are functionalized with at least one functional group configured for a click-chemistry reaction. In embodiments fluorescent dyes, fluorophores, or other dyes disclosed herein, are functionalized with at least one functional group (configured for a click-chemistry reaction) selected from the group comprising azides, alkynes, strained alkynes, alkenes, strained alkenes, tetra azides, cyclooctynes, trans-cycloctenes (TCO), phosphines, ketones, aldehydes, amines and tetrazines (Tz), enabling their conjugation to biomolecules (e.g., the present tracer compounds) via a click-chemistry reaction.

In embodiments a (fluorophore / fluorescent) dye or label is functionalized with functional group(s), suitable for a click-chemistry reaction-based conjugation / connection / ligation to a respective functional group(s) of a tracer compound (to be labeled/ conjugated/connected/ligated to the dye or label), thereby enabling a conjugation/connection/ligation of the dye or label to the tracer compound via a click-chemistry reaction.

In embodiments, the compound is 2-lminobiotinyl-L-propargylglycyl-w-lysine (2-IB-alkyne) or 2-lminobiotinyl-4-azido-L-homoalaninyl-w-lysine (2-IB-azide).

In embodiments, the tracer compound is selected from the chemical structures of 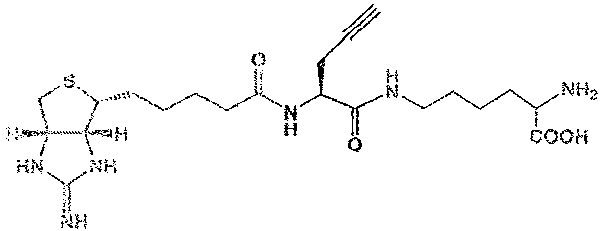 (2-lminobiotinyl-L-propargylglycyl-w-lysine (2-IB-alkyne)),
or 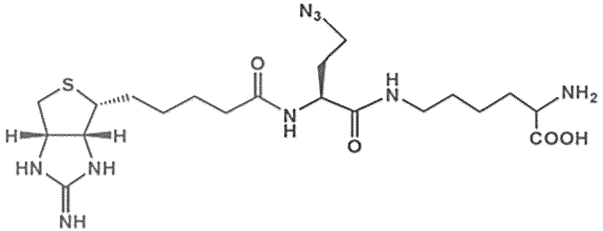 (2-lminobiotinyl-4-azido-L-homoalaninyl-w-lysine (2-IB-azide)).

In embodiments, the compound comprises a label, preferably a fluorescent dye, coupled to the connecting moiety, preferably via a triazole conjugation resulting from a click-chemistry reaction between an alkyne and an azide.

In embodiments, the compound comprises more than one connecting moiety. In embodiments compounds according to the invention comprise more than one connecting moiety, thereby enabling the linking of multiple dye molecules (via a click reaction), thereby adding a further factor of variability and flexibility to the labelling process.

In embodiments, the present tracer compound comprises more than one (optionally consecutive) connecting moiety.

In embodiments, wherein the present tracer compound comprises more than one connecting moiety, the tracer compound may comprise a structure as follows: 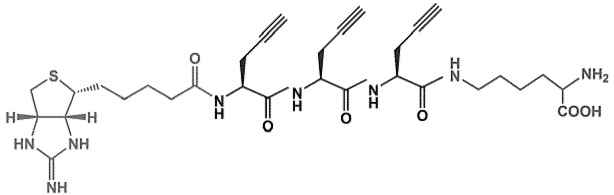 (2-IB-triple-alkyne).

In embodiments, wherein the present tracer compound comprises more than one connecting moiety, the tracer compound may be 2-IB-triple-alkyne or 2-IB-triple-azide.

In embodiments, wherein the present tracer compound comprises more than one connecting moiety, the tracer compound may be 2-IB-double-alkyne or 2-IB-double-azide.

In embodiments, wherein the present tracer compound comprises more than one connecting moiety, the tracer compound may be 2-IB-X-alkyne or 2-IB-X-azide, wherein X indicates any suitable number of the respective alkyne or azide, for example, 2-IB-double/di-alkyn or 2-IB-triple-alkyne.

In embodiments the tracer compound comprises more than one and different connecting moieties (combination of different connecting moieties), e.g., 2-IB-TCO-alkyne. Such compounds would have the advantage of enabling >3 color labelling, as they comprise suitable combinations of connecting moieties.

In embodiments the tracer compound is 2-IB-alkyne (2-lminobiotinyl-L-propargylglycyl-w-lysine, Fig. 4A) or 2-IB-azide (2-Iminobiotinyl-4-azido-L-homoalaninyl-ω-lysine, Fig. 4B). The molecules according to the invention allow, for example, multicolour fluorescent labelling of cells (e.g. neurons or glia cells; Fig. 5) together with high fidelity electrophysiological recordings (Fig. 3A). The tracer compounds according to the present invention, such as, e.g., 2-IB-alkyne and 2-IB-azide, were developed by the inventors, as there exists a limited choice of commercially available molecules for combined imaging and electrophysiological recordings and hence an unmet need of alternative electrical-neutral dyes.

In embodiments, the present tracer molecules enable to stably introduce a terminal alkyne (R-C=C-H) or azide (R-N3) functional group into living cells. In such embodiments, present molecules take advantage of recent Nobel Prize winning chemistry (Chemistry Prize, 2022; For the development of click chemistry and bioorthogonal chemistry; Carolyn R. Bertozzi, Morten Meldal and K. Barry Sharpless). Advantageously, azide and alkyne functional groups are bioorthogonal chemical reporters, meaning they do not interfere with native biochemical processes in the cell (Wang et al., 2003). Yet, both can be conjugated to reporter molecules for visualization, including for example fluorophores, via Copper(I)-catalysed Azide-Alkyne Cycloaddition (CuAAC).

In embodiments, the present compounds may be used for the labelling of cells with complex morphologies (e.g. neurons and glial cells), which can be combined with (or without) simultaneous electrical recordings and biocytin labelling.

In embodiments, the present tracer compounds lack an interaction with biocytin and/or neurobiotin labelling. In embodiments, the present tracer compounds particularly lack an interaction with biocytin and/or neurobiotin labelling at a pH below pH 9, preferably at or around pH 7.4 (physiological pH).

In embodiments, the present compounds may be used in applications where biocytin or neurobiotin are currently being used, e.g. fluorescent imaging, histology, viral infections, electron microscopy, etc.

While the present compounds may be used in embodiments for labelling of cells in combination with biocytin, it is envisioned that present compounds may be used in a variety of other research applications, for example, due to the advantageous feature of the rapidity of the click-chemistry reaction enabling conjugating azides and alkynes. The rapidity of the reaction, occurring within minutes, may lead to faster labelling experiments, which may in turn speed up experimental protocols and save time on working hours.

In embodiments, the present compounds may be used for multicolor-labelling of cells, *in vitro* and *in vivo.* As can be seen in Fig 5D the inventors have extensively tested a 3-color labelling strategy using exemplary compounds according to the invention in combination with biocytin. Even a '7-color', combinatorial labelling strategy is possible by mixing biocytin, with e.g., 2-IB-azide and 2-IB-alkyne, in varying combinations. For example, a cell may be filled with either biocytin only, 2-IB-alkyne only or 2-IB-azide only, or a combination of the dyes (e.g. biocytin + 2-IB-alkyne or all three dyes together), which would give new distinguishable color combinations. Overall, the present invention enables the mixing and combining of compounds to form greater color varieties with the potential to be widely adopted.

In embodiments, the present compounds may be provided in a solid form, such as a powder. In embodiments, the present compounds may be provided in a solid form alone or in combination with biocytin. In some of such embodiments the solid form of the present compound, e.g., powder, may be dissolved and added to an intracellular solution in preparation for injection into cells (preferably similar to solid forms of biocytin).

In embodiments, the present compounds may be provided in a liquid form, such as, e.g., a ready-made solution. In embodiments, a ready-made solution may comprise single molecules (e.g., the present compounds) at defined concentrations, or pre-defined combinations of molecules (e.g., the present compounds and biocytin and/or neurobiotin), which would facilitate ease of use in generating multi-colour combinatorial labelling.

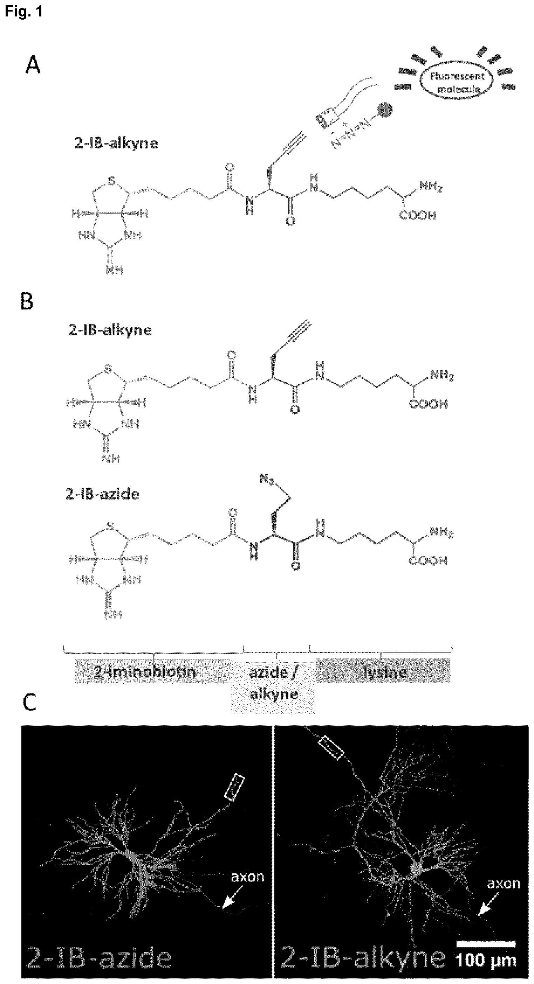

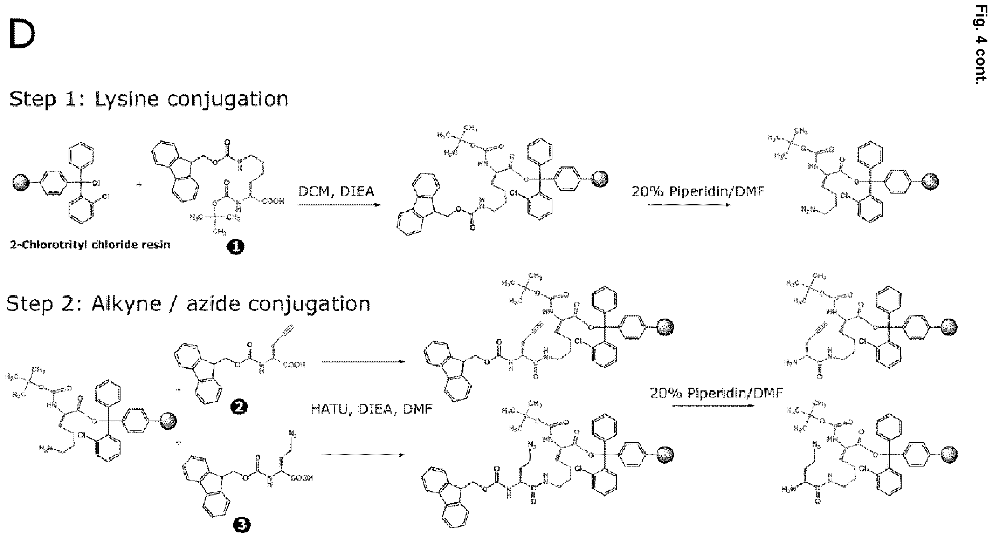

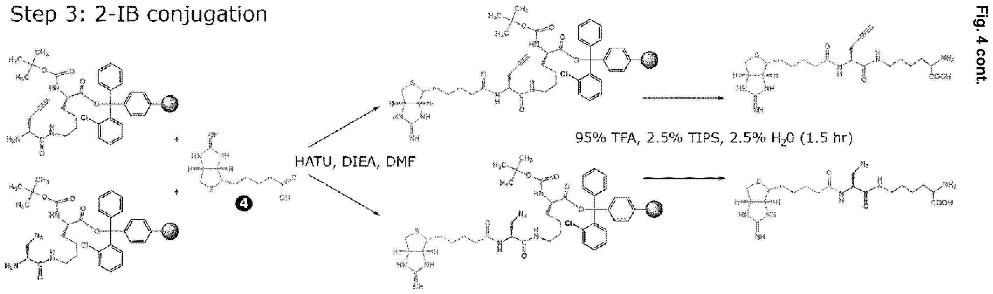

The present inventors hypothesized that filling cells with compounds comprising chemical groups capable of a click-chemistry reaction, such as azide or alkyne functional groups, should render them amenable to fluorescent labelling without interfering with their electrical properties. To test this, the present inventors developed exemplary (and non-limiting) prototype molecules according to the present invention, namely 2-IB-alkyne and 2-IB-azide. Figure 4 shows an exemplary description of the structure (A, B), components (C) and solid-phase synthesis steps (D) used to produce each of the exemplary, non-limiting molecule structures shown in Fig 1, 2 and 4.

Hence, in embodiments the present invention relates to a method for constructing a tracer compound according to the present invention (e.g., as disclosed above), comprising the coupling of:
a. a non-streptavidin-binding biotin analogue, preferably 2-iminobiotin,
b. at least one connecting moiety comprising at least one functional group configured for a click-chemistry reaction, preferably one or more of an i) azide and/or ii) alkyne moiety, and
c. a cross-linkable moiety, preferably a (para)formaldehyde cross-linkable moiety comprising an amine group,
   i. to form a tracer compound for whole-cell labelling,
d. and optionally coupling a label, preferably a fluorescent dye, to said functional group, wherein the dye is preferably coupled via a click-chemistry reaction, to form an electrophysiological neutral dye.

In embodiments, the method for constructing a tracer compound comprises solid-phase peptide synthesis.

In embodiments, the at least one functional group configured for a click-chemistry reaction of the at least one connecting moiety in b. is selected from the group comprising azides, alkynes, strained alkynes, alkenes, strained alkenes, tetra azides, cyclooctynes, trans-cycloctene (TCO), phosphines, ketones, aldehydes, amines and tetrazines (Tz).

In embodiments, the click-chemistry reaction is or comprises a Diels-Alder reaction (IEDDA), a tetrazine-trans-cyclooctene ligation, a Staudinger ligation, a strain-promoted azide-alkyne cycloaddition (SPAAC) reaction, an oxime ligation or a copper(I)-catalysed azide-alkyne cycloaddition (CuAAC).

In embodiments, the method for constructing a tracer compound according to the invention, comprising:
a. coupling a cross-linkable moiety, preferably a (para)formaldehyde cross-linkable moiety comprising an amine group, preferably lysine, to a resin, preferably in the presence of a solvent and/or a base, preferably followed by a deprotecting agent in a solvent,
b. coupling at least one connecting moiety comprising at least one functional group configured for a click-chemistry reaction, preferably selected from the group comprising azides, alkynes, strained alkynes, alkenes, strained alkenes, tetra azides, cyclooctynes, trans-cycloctene (TCO), phosphines, ketones, aldehydes, amines and tetrazines, to the resin-amino acid conjugate obtained in a.,
c. coupling of a non-streptavidin-binding biotin analogue, preferably 2-IB, to the conjugate obtained in b.

In embodiments, the method for constructing a tracer compound according to the present invention comprises in step (1) coupling a cross-linkable moiety, preferably an amino acid, e.g., lysine, preferably Boc-Lys(Fmoc)-OH, to a resin, preferably 2-Chlorotrityl chloride resin, in the presence of a solvent, preferably Dichloromethane (DCM) and a base, preferably Diisopropylethylamine (DIEA), followed by a deprotecting agent, preferably 20% Piperidine (PIP) in a solvent, preferably Dimethylformamide (DMF).

In embodiments, the method for constructing a tracer compound according to the present invention comprises in step (2) coupling at least one connecting moiety comprising at least one functional group configured for a click-chemistry reaction, preferably selected from the group comprising azides, alkynes, strained alkynes, alkenes, strained alkenes, tetra azides, cyclooctynes, trans-cycloctene (TCO), phosphines, ketones, aldehydes, amines and tetrazines (Tz), to the resin-amino acid conjugate obtained in a. by using a coupling agent, preferably 1-[Bis(dimethylamino)methylene]-1H-1 ,2,3-triazolo[4,5-b]pyridinium 3-oxid hexafluorophosphate (HATU), in the presence of a solvent, preferably DMF, and a base, preferably DIEA, followed by a deprotecting agent, preferably 20% PIP, in a solvent, preferably DMF.

In embodiments, the method for constructing a tracer compound according to the present invention comprises in step (3) coupling of a non-streptavidin-binding biotin analogue, preferably 2-IB, to the conjugate obtained in b. using a coupling agent, preferably HATU, in the presence of a solvent, preferably DMF, and base, preferably DIEA.

In embodiments, the method for constructing a tracer compound according to the present invention comprises in a step (4) cleaving the crude conjugate obtained in c. using a cleavage agent, preferably 95% Trifluoroacetic acid (TFA), together with scavenging agent, preferably 2.5% Triisopropylsilane (TIPS), and solvent / scavenger, preferably 2.5% H₂0.

In a specific embodiment, the method for constructing a tracer compound may comprise a stepwise solid-phase synthesis, e.g., using a conventional Fmoc/tBu strategy. In embodiments as solid support 2-Cl-Trityl-Harz (1.4mmol/g) from Rapp Polymere GmbH may be used and respective cross-linkable moieties, e.g., amino acids, are coupled according to standard methodologies in as single coupling with HATU as a coupling reagent. In embodiments Fmoc deprotection may be carried out with 20% piperidine in NMP and cleavage of crude conjugates from their resins may be accomplished through treatment with 95% TFA 2.5% water, 2.5% TIPS, e.g., for 1.5h. In embodiments preparative purification by high-pressure liquid chromatography (HPLC) may subsequently be carried out, e.g., on a Shimadzu LC-8A system with a Kromasil 100, C18 (245x50mm) column (e.g., with buffer A: 0.2% TFA in water, buffer B: 0.2% TFA in water:acetonitrile, 1:4).

In embodiments, the non-streptavidin-binding biotin analogue may exhibit a nucleic acid and/or amino acid sequence identity and/or structural identity with biotin, 2-iminobiotin, biocytin and/or neurobiotin of at least 70 % or higher.

In embodiments, the non-streptavidin-binding biotin analogue may exhibit a sequence (or structural) identity with biotin of 45, 50, 55, 60, 65, 70, 75, 80, 81, 82, 83, 84, 85, 86, 87, 88, 89, 90, 91, 92, 93, 94, 95, 96, 97, 98 or 99%, or a sequence identity of at least 70 % or higher.

In embodiments, the non-streptavidin-binding biotin analogue may exhibit a sequence (or structural) identity with biocytin and/or neurobiotin of 45, 50, 55, 60, 65, 70, 75, 80, 81, 82, 83, 84, 85, 86, 87, 88, 89, 90, 91, 92, 93, 94, 95, 96, 97, 98 or 99%, or a sequence identity of at least 70 % or higher.

In embodiments, the non-streptavidin-binding biotin analogue may exhibit a sequence (or structural) identity with 2-iminobiotin of 45, 50, 55, 60, 65, 70, 75, 80, 81, 82, 83, 84, 85, 86, 87, 88, 89, 90, 91, 92, 93, 94, 95, 96, 97, 98 or 99%, or a sequence identity of at least 70 % or higher.

In another aspect the present invention relates to a method for labelling a cell, comprising:
a. injecting a tracer compound according to the present invention into the cell, preferably using a glass micro pipette filled with an intracellular solution, preferably using the "whole-cell patch-clamp" technique, preferably allowing passive diffusion, or by applying a current, or by applying the compound juxtacellularly (near or besides, external to the cell),
b. optionally, during which, determining electrophysiological properties (performing electrophysiology measurements) of the cell, preferably by establishing voltage differences between the inside and the outside of the cell, to measure current flow and resistance across the membrane of the cell, or to passively record voltage differences between the inside and the outside of the cell, to measure the amplitude, frequency and duration of spontaneous current flow across the membrane of the cell,
c. allowing the tracer compound to diffuse through and/or around the cell, preferably for at least 15 minutes,
d. fixing the cell and tracer compound with a crosslinker, preferably a formaldehyde, more preferably 4% paraformaldehyde, preferably for at least 1 hour,
e. optionally permeabilizing the cell, preferably with a surfactant, preferably with 0.1-0.5% Triton-X 100,
f. adding a label into the cell, or juxtacellularly, that reacts with and becomes coupled to the functional group of the connecting moiety of the tracer compound, preferably connecting azide and alkyne function groups, preferably using a catalyst, preferably Copper(I), preferably in solution,
g. detecting and imaging the label and/or the cell.

In embodiments, a tracer compound according to the present invention may be injected into a cell either (i) using a glass micro pipette filled with an intracellular solution, preferably using the "whole-cell patch-clamp" technique, and preferably allowing passive diffusion, or (ii) by applying a current, and/or (iii) by applying the compound juxtacellularly (near or besides, externally to the cell).

In embodiments, a tracer compound according to the present invention may be injected into a cell through intracellular recording techniques.

In embodiments, determining electrophysiological properties (performing electrophysiology measurements) of a cell may comprise establishing voltage differences between the inside and the outside of the cell, preferably to measure current flow and resistance across the membrane of the cell, and/or to passively record voltage differences between the inside and outside of the cell, to measure the amplitude, frequency and duration of spontaneous current flow across the membrane of the cell.

In embodiments, electrophysiological properties that are determined, comprise the voltage responses to hyperpolarizing and depolarizing current pulse, or the recordings of the inhibitory or excitatory postsynaptic current.

In embodiments of fixing (fixation) the cell and tracer compound, the crosslinker is a formaldehyde, preferably paraformaldehyde with a concentration of between 0.5-10% or 1-5%, more preferably 1, 2, 3, 4, 5, 6, 7, 8, 9 or 10% paraformaldehyde or 4% paraformaldehyde.

In embodiments, a cell is labelled by adding a label or dye into the cell or juxtacellularly to the cell. In embodiments, the label or dye reacts with and becomes coupled to the functional group of the connecting moiety of the tracer compound comprised within (as previously added to) the cell. In embodiments, the label comprises at least one functional group configured for a click-chemistry reaction, preferably selected from the group comprising azides, alkynes, strained alkynes, alkenes, strained alkenes, tetra azides, cyclooctynes, trans-cycloctene (TCO), phosphines, ketones, aldehydes, amines and tetrazines (Tz), which is capable of connecting/coupling the label with the tracer compound according to the invention, optionally using a catalyst (e.g., such as Copper(I)).

In embodiments, the fluorescent dye or label is functionalized with functional group(s) suitable for the respective functional group(s) of a tracer compound to be labeled/coupled/conjugated/connected/ligated to the dye or label, thereby enabling a coupling/conjugation/connection/ligation of the dye or label to the tracer compound via a click-chemistry reaction.

In embodiments, a tracer compound according to the present invention is injected into the cell at a concentration between 1 and 10 mM. In specific embodiments, a tracer compound according to the present invention is injected into the cell at a concentration of 2.69 mM, 3 mM or 6 mM. In embodiments, a tracer compound according to the present invention is injected into the cell at a concentration of 1 mM, 2 mM, 2.5 mM, 2.7 mM, 3 mM, 4 mM, 5 mM, 5.5 mM, 6 mM, 6.5 mM, 7 mM, 8 mM, 9 mM, 10, mM, 12, mM, 15 mM or20 mM. In embodiments, injecting a tracer compound according to the present invention into the cell is achieved by/comprises techniques such as dissolving the tracer compound into a suitable intracellular solution, preferably potassium based, preferably containing: 130 mM potassium gluconate, 0.2 mM EGTA, 10 mM KCI, 10 mM HEPES, 2 mM MgCl₂, 2 mM NA₂ATP, 0.3 mM Na₂GTP, 1 mM Na₂Creatine;); pH 7.3, before penetrating the membrane of the cell and allowing for diffusion.

In embodiments a tracer compound according to the present invention is injected into the cell using whole-cell patch-clamp recordings.

In non-limiting, exemplary embodiments this involves the recording of cells, e.g., of single neurons, e.g., using a Multiclamp 700B amplifier (Molecular Devices), and recording electrodes (3-5 MΩ), preferably pulled from borosilicate glasses with filament (outer diameter, 2 mm; wall thickness, 0.5 mm). In embodiments, a glass pipette containing the tracer compound may be tightly sealed onto the cell membrane. In embodiments, the membrane potential of the cell is measured and compared to the command potential (-65mV). In embodiments, if there are differences between the command potential and the measurement, a current will be injected.

In embodiments, for the measurement of intrinsic property, voltage responses may be evoked by hyperpolarizing and depolarizing current pulses (e.g., 500 ms, -250 - +250 pA and step size: +50 pA).

In embodiments, the access to the intracellular compartment of a cell may be achieved using a glass micropipette. In embodiments a heated glass micropipette, pulled from borosilicate glass, and filled with suitable intracellular solution, with a tip resistance of 3-5 MΩ is used. In embodiments, the pipette is positioned and gentle negative pressure applied to form a Giga-Ohm seal on the membrane of the cell and then rupturing the membrane below the tip, e.g. by applying sharp negative pressure, to gain access to the intracellular space of the cell.

In specific embodiments, determining electrophysiological properties (performing electrophysiology measurements) of the cell comprises, e.g., injecting current pulses (for example 500 ms pulse duration, range -250 to +250pA and step size, with +50 pA incremental steps) to test subthreshold and suprathreshold electric behavior of the cells. In embodiments, suprathreshold pulses generate action potential firing to measure the initiation, amplitude, frequency and waveform properties of action potentials, the latter preferably tested at or near to the cells resting membrane potential.

In embodiments of the method for labelling a cell, the method steps prior to fixing the cell are performed at a physiological pH, preferably below pH 9, more preferably between pH 4.5 to 8.0, or pH 6.8 and 7.4.

In embodiments, the pH before fixing cells with (para)formaldehyde, e.g., during the electrophysiological recording and/or injection/introduction of the tracer compound into the cell, may be typically maintained at the physiological pH for cells, e.g., neurons, preferably around pH 7.4, to ensure the viability and physiological functioning of the cell (to reduce impairment of physiological processes).

In embodiments, detecting and imaging the label and/or the cell comprises imaging methods such as microscopy, preferably fluorescence microscopy and/or confocal microscopy, and/or brightfield microscopy when combined with histological staining methods, e.g. DAB-staining.

In embodiments, the pH after fixing cells and/or optional permeabilization of the cell is preferably maintained at the physiological pH for cells, preferably around pH 7.4 (unless there is a specific reason).

In embodiments the physiological pH of preferably around pH 7.4 has the additional advantage that a binding between 2-IB and streptavidin is prevented. At very high pH values of pH > 9-11 a binding of 2-IB to streptavidin may occur with a higher likelihood, such that parallel differential labelling of neighboring cells with the present compounds and biocytin may be impaired. However, in certain embodiments, e.g., when no parallel biocytin labeling is performed, a pH over 7.4 may be desired or advantageous (Athappilly et al., 1997).

In embodiments of the method for labelling a cell, the cell is an electrically-active cell, preferably a neuron, a glial cell, or muscle cell, preferably a cell of the CNS and PNS, or any other cell type possessing ion channels, an electrochemical potential, or electrical activity.

In embodiments of the method for labelling a cell, biocytin and/or neurobiotin are additionally injected into the cell or nearby cell, or applied juxtacellularly, and an additional label coupled to streptavidin is added to the cell, and subsequently detected after binding to biocytin and/or neurobiotin.

In another aspect the present invention relates to a kit for carrying out the method for labelling a cell according to the invention, comprising:
a. at least one tracer compound according to the present invention, and
b. one or more labels (preferably comprising a fluorescent dye) comprising at least one functional group configured for the corresponding click-chemistry reaction,
c. and optionally one or more of biocytin or neurobiotin, a label (preferably comprising a fluorescent dye) coupled to streptavidin, a crosslinker (preferably (para)formaldehyde), a cell-permeabilizing agent (such as a detergent), suitable buffers, and reagents for performing a click-chemistry reaction, preferably copper(II) sulfate, sodium ascorbate and Tris(benzyltriazolylmethyl)amine (THPTA).

In embodiments, the present compounds may be provided in a solid form, such as a powder. In embodiments, the present compounds may be provided in a solid form alone or in combination with biocytin. In some of such embodiments the solid form of the present compound, e.g., powder, may be dissolved and added to an intracellular solution in preparation for injection into cells (preferably similar to solid forms of biocytin).

Embodiments and features of the invention described with respect to the peptide conjugates, the method and kits are considered to be disclosed with respect to each and every other aspect of the disclosure, such that features characterizing the peptide conjugates may be employed to characterize the methods, or kit and vice-versa. The various aspects of the invention are unified by, benefit from, are based on and/or are linked by the common and surprising finding of the superior and electrically neutral labelling capabilities of the present tracer compounds.

### DETAILED DESCRIPTION OF THE INVENTION

All cited documents of the patent and non-patent literature are hereby incorporated by reference in their entirety.

The present invention is directed to a tracer compound enabling the labelling or detection of preferably electrically-active cells, such as neurons, glia or muscle cells, by diffusing within the cell in a way allowing the detection of even intricate parts of the cell, such as cellular protrusions, and preferably without impairing the electrophysiological properties and maintaining the viability of the cell.

In the context of the present invention the term `electrical properties' of a cell or `electrophysiological properties' refers to a cells ability to generate and respond to changes in potential across its out membrane ("membrane potential"), primarily determined by the flow of ions through ion channels traversing the plasma membrane and the capacitive property of the membrane.

Common methods for measuring the electrophysiological properties of a cell are the patch-clamp and sharp electrode techniques. Common methods for measuring the electrophysiological properties of a cell are whole-cell patch-clamp. It is a powerful method and is essential for understanding cellular electrical activity and the behavior of ion channels, providing detailed insights into cellular function used. For the patch-clamp technique, several configurations exist, including whole-cell mode, as described, whereby the cell is recorded from intracellularly; cell-attached mode, whereby a Giga-Ohm seal is formed against the membrane, but the membrane is not penetrated; perforated-patch mode, whereby cell-attached mode is established, but in combination with a dissolved perforating agent in the intracellular solution, such as gramicidin, which creates small pores through the membrane of the cell to allow limited electrical accessibility. In embodiments, biocytin can be 'iontophorized' into the cells with electric pulse. Alternatively, the sharp electrode technique, which forgoes establishing a Giga-Ohm seal, and simply aims to impale the cell to be able to record from intracellularly.

One of the most important ways of studying the electrical properties of neurons is through intracellular recordings, such as the `whole-cell patch-clamp' technique (Hamill et al., 1981). The technique involves recording a neuron's electrical activity from the inside, using a glass micropipette, filled with a suitable electrolyte solution and an electrode coupled to an amplifier. It is a widely used technique and is the gold standard for recording the synaptic inputs and intrinsic activity of neurons. The technique benefits from both a high sensitivity and temporal resolution and has been widely adopted, being used by thousands of research institutions across the globe. An advantage of the method is that it can be easily combined with the intracellular labelling of the recorded cell. Alternatively, the sharp electrode technique, which uses finer, higher resistance electrodes and simply aims to impale the cell to be able to record from intracellularly.

A 'conjugate' refers in the context of the present invention to a molecule being linked or conjugated to another moiety, such as a label and/or a connecting moiety. In embodiments a conjugate may be a molecule comprising different functional groups or moieties. The linking of different molecule moieties may in embodiments be a direct covalent linkage to the other moiety, e.g., label, or can comprise a linking moiety or linker in between two of the conjugate's functional groups or moieties. The person skilled in the art is aware of different techniques to link molecules to each other to form conjugates. An exemplary and non-limiting reaction for linking or construction the present compounds or conjugates is shown in Figure 4.

In the context of the present invention the term `tracer compound' preferably refers to a compound that may be used for labelling and/or detecting cells, for example, by being introduced into a cell and previously or subsequently being linked to a label thereby enabling the labelling and detection of a cell, and, for example, its location, movements and/or physiological properties, such as shape and/or protrusions. In embodiments the terms `tracer compound', `tracer', 'compound', 'conjugate' or `tracer conjugate' may be used interchangeably.

Herein, a `connecting moiety' may also be referred to as `conjugation moiety' or `linking moiety' referring to a moiety of the present tracer compounds that enables the linking of, e.g., other molecules to the present compounds. Non-limiting examples of other molecules or moieties that may be linked, connected or conjugated via a connecting moiety are dyes or fluorophores, molecules comprising dyes or fluorophores or other molecules, preferably enabling the labelling and/or detection of cells.

In embodiments, the connecting moiety comprises at least one functional group configured for click chemistry reaction, such as a group selected from azides, alkynes, tetra azides, trans-cycloctene (TCO) and tetrazines, that preferably enables the generation of a triazole conjugation. In embodiments the connecting moiety thereby enables the connection, conjugation or linkage of a dye or fluorophore to the tracer compound via a click chemistry reaction, e.g., forming a triazole conjugation. In other words, in embodiments, the present compounds comprise a label, preferably a dye or fluorophore, coupled to the connecting moiety, preferably via a triazole conjugation resulting from a click-chemistry reaction between an alkyne and an azide. In other embodiments a dye or fluorophore may be linked to the connecting moiety via a click chemistry reaction involving azides, alkynes, tetra azides, trans-cycloctene (TCO), tetrazines, strained alkynes, alkenes, strained alkenes, cyclooctynes, phosphines, ketones, aldehydes, amines and tetrazines.

A `click reaction' (`click chemistry') refers to a linking reaction joining a substrate of choice with specific biomolecules, such as a biomolecule and a reporter molecule or label. Click reactions are defined by the use of mild reaction conditions (ambient temperature and pressure) in and compatible with water as a solvent. As such, they can be useful in the detection, localization and qualification of biomolecules. In chemical synthesis, click chemistry is a class of biocompatible small molecule reactions commonly used in bioconjugation. Click chemistry commonly is not limited to a single specific reaction but refers to a mode of generating products that follow examples in nature by joining small modular units together. However, click chemistry is not limited to biological conditions. In embodiments, the at least one functional group configured for a click-chemistry reaction is selected from the group comprising azides, alkynes, tetra azides, trans-cycloctene (TCO) and tetrazines (examples are depicted in Figure 3B), and preferably enables the generation of a triazole conjugation. In embodiments, the present tracer molecules enable to stably introduce, e.g., a terminal alkyne (R-C=C-H) or azide (R-N3) functional group into living cells. In such embodiments, the present invention employs the advantageous mechanism of click-chemistry reaction, wherein, e.g., azide and alkyne functional groups are used as biorthogonal chemical reporters, not interfering with native biochemical processes in the cell. Still, in embodiments herein any of said functional groups may be selected from the group comprising azides, alkynes, tetra azides, trans-cycloctene (TCO), tetrazines strained alkynes, alkenes, strained alkenes, cyclooctynes, phosphines, ketones, aldehydes, amines and tetrazines can be conjugated to reporter molecules, including for example fluorophores, via a click-chemistry reaction. In embodiments, click-chemistry reactions may comprise a Diels-Alder reaction (IEDDA), a tetrazine-trans-cyclooctene ligation, a Staudinger ligation, a strain-promoted azide-alkyne cycloaddition (SPAAC) reaction, an oxime ligation or a copper(I)-catalysed azide-alkyne cycloaddition (CuAAC).

'Covalently linked' refers herein to the linkage of two molecules (e.g., an amino acid sequence and a label) through a covalent bond. A covalent bond is a chemical bond in which electrons are exchanged to form electron pairs between atoms, in chemistry it refers to the interatomic bond formed by the sharing of a pair of electrons between two atoms. This bond is formed by the electrostatic attraction of their nuclei to the same electrons. A covalent bond occurs when the bonded atoms have a lower total energy than that of widely separated atoms.

In some embodiments, a ligand binds specifically to its target. `Specific binding' is to be understood as via one skilled in the art, whereby the skilled person is clearly aware of various experimental procedures that can be used to test binding and binding specificity. Methods for determining equilibrium association or equilibrium dissociation constants are known in the art.

Herein a `(para)formaldehyde cross-linkable group' preferably comprises an amine group thereby enabling the crosslinking by formaldehyde or paraformaldehyde. Examples of compounds or molecules comprising an amine group are amino acids. In some preferred embodiments the amino acid is lysine. Hence, in embodiments the term `cross-linkable group' refers to molecules comprising an amine group, such as amino acids or analogous or derivatives thereof, as said molecules enable a cross-linking (may be cross-linked) by formaldehyde or paraformaldehyde. In embodiments the crosslinking with (para)formaldehyde enables the fixation of the cell comprising or together with the present tracer compound for subsequent histological processing. In general (para)formaldehyde induces the cross-linking of proteins within a cell, primarily through cross-linking lysine residues. Hence, in embodiments for subsequent detection and analysis, the present compounds may be fixed by (para)formaldehyde to (all) other cross-linkable components (e.g., proteins and peptides) of a cell, into which the present compound has been introduced. In embodiments the crosslinking with (para)formaldehyde enables the fixation of tissues and cells, preserving the location and structure of proteins, nucleic acid, and other macromolecules within cells, and comprising the present tracer compound for subsequent histological processing.

In general (para)formaldehyde induces the cross-linking of proteins within a cell, primarily through the amino groups (-NH₂) of lysine residues. Hence, in embodiments for subsequent detection and analysis, the present compounds containing the amino groups (-NH₂) may be fixated by (para)formaldehyde along with all other cross-linkable components (e.g., proteins and peptides) of a cell, into which the present compound has been introduced. Crosslinking preserves the proteins in place, preventing them from moving or interacting with other molecules, which could otherwise lead to artefacts in microscopy or other analyses.

Herein a `non-streptavidin-binding biotin analogue' refers to a molecule enabling the tracer compound to diffuse within a biological cell with essentially the same properties as biocytin and/or neurobiotin. Such molecules may be structurally (regarding its chemical structure) related (analogous or derivatives thereof) or un-related to biotin. Herein it is particularly preferred that said molecules cannot be bound by streptavidin. In embodiments the present tracer compounds may be used for labelling cells in parallel to biocytin or neurobiotin, wherein a differential labelling of cells by the present tracer compounds and biocytin or neurobiotin is preferably enabled as biocytin or neurobiotin may be detected via a streptavidin-coupled dye, while the present tracer compounds may be detected by a different (non-streptavidin coupled) dye. In embodiment the detection of the present tracer compounds may be achieved by a fluorophore or dye being connected via a click-chemistry reaction to the present tracer compounds. Herein, a non-streptavidin-binding biotin analogue may be a reversible-streptavidin biotin analogue, such as a molecule enabling the tracer compound to diffuse within a biological cell with essentially the same properties as biocytin and/or neurobiotin, and with none, a low or insignificant binding to streptavidin.

Hence, while a 'dye' or `reporter molecule' may preferably not be linked via biotin-streptavidin coupling to the present tracer compounds, the present tracer compounds preferably still comprise a group, structure or molecule enabling the diffusion of the tracer within the cell in a similar manner to biocytin or neurobiotin, thereby ensuring a comparability of the present tracer compounds, and the cellular labelling achieved therewith, with biocytin or neurobiotin (labelling). In embodiments, the present compounds may therefore comprise a `non-streptavidin-binding biotin analogue', such as the biotin analogue 2-iminobiotin (2-IB) that shows no relevant streptavidin binding, preferably at or near neutral pH levels.

Herein a 'sample' may be any relevant sample for analysis, such as (without limitation) a sample taken from a patient, a cell culture of patient cells or cell lines, an animal, or a cell culture of animal cells or cell lines of a biopsy, a blood sample, a tissue sample, an environmental sample, or a food-derived sample. A sample may be any kind of biological sample. As used herein, the term 'sample' is preferably a biological sample that is obtained or isolated from a patient, a subject, an animal, the environment or any other biological source. In some embodiments a sample may refer to a sample of bodily tissue or fluid, such as a tissue biopsy, e.g., from neural or muscle tissue, cerebrospinal fluid, a liquid biopsy, blood, serum, plasma, urine, saliva, sputum, pleural effusions, cells, a cellular extract, a tissue sample, a tissue biopsy, an organ or a fraction thereof, such as a brain or a section thereof, neural tissue or muscle tissue, muscle or a section thereof, a stool sample and the like.

Herein a "subject", may be an organism, a cell culture of a subject's cells or cell lines derived therefrom, an animal, or a cell culture of animal cells or cell lines. Herein a subject refers to a species from which a sample is taken, and/or whose biological material makes up the majority of the biological material of a sample. A subject or patient can be selected from the group comprising vertebrae, animals, livestock, mammals, animal models, rodents, humans, preferably mammal or human.

'Neurons' (nerve cells) or `cells of neuronal tissue' refer herein to cells of the nervous system. Cells of the nervous system commonly comprise neurons and neuroglia, or glial cells. Neurons commonly are able to conduct or transmit electrical impulses/signals. Neurons commonly comprise three parts, namely a cell body (soma), one or more dendrites, and one axon, wherein dendrites and axons are cytoplasmic extensions, or processes, that protrude from the soma. Axon collaterals are branches of axon that again terminate in many short branches (telodendria) that are able to form synaptic bulbs or terminals. Glia cells or neuroglia are cells that provide a support system for the neurons. Commonly axons comprise a segmented sheath of myelin, with unmyelinated regions between the segments, called nodes, however, in the CNS many axons are not myelinated.

It is generally considered that also glial cells comprise ion channels, show changes in membrane potential, and can conduct calcium signals. However, glial cells do not have action potentials, as e.g., neurons have.

Neurons, for example, are known to be able to form `cellular protrusions'. In neurons, for example, protrusions may serve to communicate with other neurons, by forming synapses. In the context of the present invention, cellular protrusions may refer, without limitation thereto, e.g., to axonal collaterals and dendritic spines. In general, cellular protrusions are outward extensions of the plasma membrane that enable the sensing of the general environment of a cell and interaction and communication with other cells.

Herein, `cells of the central nervous system (CNS)' may preferably comprise, without being limited thereto, neurons and glial cells, the glial cells preferably comprising astrocytes, oligodendrocytes, ependymal cells, radial glial cells and/or microglial cells (wherein astrocytes are present in the brain and spinal cord). In general, neurons are the cells of the CNS capable of transmitting electrochemical signals (comprising electrophysiological properties), whereas glial cells commonly support and insulate neurons.

Herein, `cells of the peripheral nervous system (PNS)' may preferably comprise, without being limited thereto, neurons, Schwann cells and satellite cells. Commonly the PNS is defined as nervous tissue located outside of the brain and spinal cord. As in the CNS neurons of the PNS are capable of transmitting electrochemical signals (comprising electrophysiological properties), while Schwann cells and satellite cells serve to support and insulate the neurons, as glia cells, their CNS counterparts.

`Amino acids' (AA, aa) are chemical compounds with a nitrogen (N) containing amino group and a carbon (C) and oxygen (O) containing carboxylic acid group. The class of amino acids includes organic compounds that contain at least one amino group (-NH₂ or substituted -NR₂) and one carboxy group (-COOH) as functional groups. Selected α-amino acids are the natural building blocks of proteins, which are linked together to form chains by the carboxy group of one amino acid forming a peptide bond with the amino group of the next. Amino acids linked to form a polymer differ in their side chains and together determine the shape with which the polypeptide then unfolds in the aqueous environment to form the native protein.

A `peptide' is an organic compound containing peptide bonds between amino acids. Accordingly, a `peptide' or `peptide sequence' refers to the sequence (and/or identity) of consecutively linked amino acids of a peptide. According to their number, oligopeptides with few are distinguished from polypeptides with many amino acids. Long polypeptide chains are also called `proteins', especially those formed by protein biosynthesis. As used herein, 'polypeptide' shall mean both peptides and proteins. Herein, any polypeptides may be naturally occurring or recombinant (i.e., produced via established molecular biology techniques), and may contain mutations (e.g., point, deletion and insertion mutations) as well as other covalent, non-proteinogenic amino acids, artificial amino acids and/or other molecular bonds to additional components.

Amino acid sequence variants of the claimed compounds, conjugates, proteins and/or other biomolecules, for example defined by the claimed % sequence identity, that maintain the said properties of the invention, are also included in the scope of the invention. Such variants, which show alternative sequences, but maintain essentially the same physiological, chemical, physical or biological properties, such as diffusion properties, as the specific compounds or (bio)molecules provided are known as (functional) analogues, or as (functional) derivates. Sequence identity relates to the percentage of identical nucleotides or amino acids when carrying out a sequence alignment.

Protein sequence modifications or variants may occur through deletions, additions and substitutions. Substitutions as defined herein are modifications made to the amino acid sequence of a peptide or protein, whereby one or more amino acids are replaced with the same number of (different) amino acids. Additions, deletions and substitutions may be natural or artificial. It is well known in the art that amino acid substitutions may be made with or without significantly altering a protein's function.

For example, the non-polar amino acids Gly, Ala, Val, Ile and Leu; the non-polar aromatic amino acids Phe, Trp and Tyr; the neutral polar amino acids Ser, Thr, Gln, Cys, Asn and Met; the positively charged amino acids Lys, Arg and His; the negatively charged amino acids Asp and Glu, are groups of conservative amino acids.

| **Original residue** | **Preferred conservative substitutions** | **Examples of exemplary substitutions** |
|---|---|---|
| Ala (A) | Val | Val; Leu; Ile; selenocysteine |
| Asg (R) | Lys | Lys; Gln; Asn |
| Asn (N) | Gln | Gln; His; Asp, Lys; Arg |
| Asp (D) | Glu | Glu; Asn |
| Cys (C) | Ser | Ser; Ala |
| Gln (Q) | Asn | Asn, Glu |
| Glu (E) | Asp | Asp; Gln |
| Gly (G) | Ala | Ala |
| His (H) | Arg | Asn; Gln; Lys; Arg |
| Ile (I) | Leu | Leu; Val; Met; Ala; Phe; Norleucine |
| Leu (L) | Ile | Norleucine; Ile; Val; Met; Ala; Phe |
| Lys (K) | Arg | Arg; Gln; Asn |
| Met (M) | Leu | Leu; Phe; Ile |
| Phe (F) | Tyr | Leu; Val; Ile; Ala; Tyr |
| Pro (P) | Ala | Ala |
| Ser (S) | Thr | Thr |
| Thr (T) | Ser | Ser |
| Trp (W) | Tyr | Tyr; Phe |
| Tyr (Y) | Phe | Trp; Phe; Thr; Ser |
| Val (V) | Leu | Ile; Leu; Met; Phe; Ala; Norleucine |

'Variants' of proteins or peptides, as defined in the context of the present invention, may be generated that have an amino acid sequence that differs from the original sequence in one or more mutation(s), such as one or more substituted, inserted and/or deleted amino acid(s). In embodiments, any sequence variation described herein with respect to percentage identity, may apply to any one or more embodiments, described throughout the application as a whole.

Fragments of proteins or peptides in the context of the present invention may typically comprise a sequence of a protein or peptide as defined herein, that is, with respect to its amino acid sequence (or its encoded nucleic acid) molecule, N-terminally and/or C-terminally truncated compared to the amino acid sequence of the original (native) protein (or its encoded nucleic acid molecule).

In embodiments, a derivate, analogues, variant or fragment of a protein may typically comprise an amino acid sequence having a sequence identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98%, 99%, or 99.5% preferably of at least 70%, or at least 80%, with an amino acid sequence of the respective naturally occurring full-length protein.

In embodiments, a derivate, analogue, or variant of a compound or molecule may typically comprise a chemical structure with an identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98%, 99%, or 99.5% preferably of at least 70%, or at least 80%, with a respective other compound or molecule.

It is understood that derivates, analogues and variants of compounds, molecules and proteins described herein can be selected by one of ordinary skill in the art to provide compounds that are chemically stable and that can be readily synthesized by techniques known in the art and further by the methods set forth in this disclosure. Various ways of preparing functionally analogous molecules, compounds, proteins or peptides have been disclosed in the prior art and are known to a skilled person.

Herein, a "label" or "dye" or "reporter molecule" is preferably any molecule, compound, substance, atom, chemical modification or any other entity or structure enabling possible the detection of the tracer compound and/or the cell stained / filled therewith. In embodiments, the label may be considered a "dye" that facilitates the detection of a cell and/or parts and/or structures of a cell. In embodiments, a "label" comprises a fluorescent or luminescent label or dye molecule. In general, fluorescence is considered as a form of luminescence occurring when matter emits light of a certain wavelength, e.g., after absorbing electromagnetic radiation. In some embodiments the terms "label" or "dye" or "reporter molecule" refer to a fluorophore, fluorescent or luminescent dye or label and said terms may be used interchangeably.

A "fluorophore" (or fluorochrome, or chromophore) is a fluorescent chemical compound capable of re-emiting light upon excitation. Fluorophores or fluorescent or luminescent labels or dyes include, without being limited thereto, rhodamine and derivatives, Rhodamine Green, Oregon Green 30 488, Oregon Green 514, 7-NH2-4CH3-25-coumarin-3-acetate (AMCA), lissamine fluorescein, 5-bromomethylfluorescein and derivatives, DAPI, Hoechst 33258, R-phycocyanin, B-phycoerythrin, R-phycoerythrin, Lucifer Yellow, IAEDANS, 7-Me2N-coumarin -4-acetate, 7-OH-4-CH3-coumarin-3-acetate, monobromobiman, Pyrene trisulfonates such as Cascade Blue and monobromotrimethyl ammoniobiman, Texas Red, FAM, TET, CAL Fluor Gold 540, JOE, VIC, Quasar 570, Cal Orange, CAL Fluor Orange 560, Cy3, Cy3.5, Cy5, Cy5.5, NED, Oyster556, TMR, BODIPY TMR-X, Quasar-570, CAL Fluor Red 590, HEX, ROX, LC Red 610, CAL Fluor Red 610, CAL Fluor Red 635, LC Red 640, Oyster645, LC Red 670, Quasar670, LC Red 705, BODIPY FL, Cal Gold, BODIPY R6Gj, Yakima Yellow, JOE, HEXTAMRA, Rhodamine Red-X, Redmond Red, BODIPY 581/591, Cal Red/Texas Red, BODIPY 630/665-X, BODIPY TR-X, Quasar-670/Cy5, Pulsar-650, Atto-488, Atto532, Atto-Rho-6G, Atto-Rho101, Atto-647N, Alexa Fluor 647, Atto-680, BMN-488, BMN-505, BMN-536, BMN-562, Dy590, Dy490, Dy636, Dy682 and DyLight 650-Phosphine.

Fluorescence imaging comprises various techniques for the visualization of fluorescent labels or fluorescence dyes as markers of biological structures and functions. In general, fluorescence imaging enables the detection, monitoring and analysis of, for example, cellular shapes, cellular protrusions, cellular movement, cellular interaction, molecular functions and other cellular processes in the cellular and tissue context. Fluorescence detection or fluorescence imaging comprises techniques such as, without limitation thereto, microscopy, confocal microscopy, fluorescence activated cell sorting (FACS), and spectroscopy. Commonly, during fluorescence microscopy the specimen is illuminated with a specific wavelength(s) of light, which is then absorbed by the fluorophore(s) and light of longer wavelengths is emitted from the fluorophore(s). In embodiments, in the microscope a spectral emission filter separates the excitation light from the fluorescence emitted from the specimen, commonly having a weaker signal intensity.

### FIGURES

The invention is further described by the following figures. These are not intended to limit the scope of the invention but represent preferred embodiments of aspects of the invention provided for greater illustration of the invention described herein.
**Figure 1****: A.** Principle of click chemistry reaction to link a label, such as a fluorescent molecule, to a connecting moiety of an embodiment of the present invention. **B.** Structure of the exemplary embodiments 2-lminobiotinyl-L-propargylglycyl-w-lysine (2-IB-alkyne) or 2-Iminobiotinyl-4-azido-L-homoalaninyl-w-lysine (2-IB-azide). **C.** Example experiment showing successful labelling of cells with two compounds according to the invention, namely 2-IB-azide and 2-IB-alkyne, enabling microscopic detection of the entire cell body including cellular protrusions, such as dendrites, dendritic spines and axons.

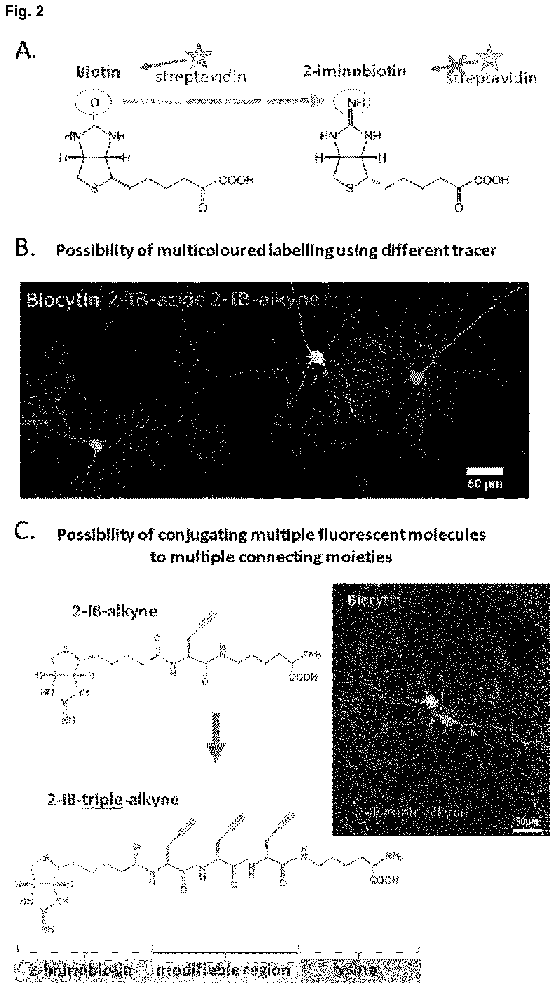

**Figure 2****: A.** Biotin enables the linking of a streptavidin-coupled label, whereas 2-imminobiotin (2-IB) is not bound by streptavidin. **B.** In embodiments the use of a non-streptavidin-binding biotin analogue, e.g., 2-IB, in the present compounds enables the parallel and non-overlapping labelling of neighboring cells with different fluorescent dyes, as distinct reporter dyes may either be coupled to biotin (via streptavidin) or via a click-chemistry reaction to a connecting moiety of a compound according to the present invention. C. Image of stained mouse hippocampus. In embodiments compounds according to the invention comprise more than one connecting moiety, thereby enabling the linking of multiple dye molecules and adding a further factor of variability and flexibility to the labelling process.
**Figure 3****: A.** The first row shows patch clamp recordings evidencing that the present compounds, just as biocytin, are electrophysiologically-neutral when applied (e.g., injected to) to a living cell. The recording shows no impairment on the electrophysiological properties (intrinsic electrical properties) of the injected cell through the compounds according to the present invention, also in comparison to the established prior art molecule biocytin. The voltage responses of the recorded and filled neurons to hyperpolarizing and depolarizing current pulses (duration: 500 ms. From -250 pA to 0 pA, 50 pA steps. Suprathreshold pulse: 250 pA). The second row shows summary violin plots of the resting membrane potential (RMP), input resistance (Rin), rheobase, AP peak from threshold and AP half-height width measurements made from filled neurons. Note that there were no significant differences observed between biocytin filled cells and either 2-IB-azide or2-IB-alkyne filled cells. Statistics: Kruskal-Wallis non-parametric comparison with Dunn's multiple comparison testing. (Number of neurons investigated: Biocytin n = 31, 2-IB-azide n = 33 and 2-IB-alkyne n = 34). **B.** The connecting moiety may comprise at least one functional group configured for a click-chemistry reaction selected from alkynes (III), tetra azides (N₃), tetrazines (Tz) and trans-cycloctene (TCO).
**Figure 4****:** Solid-phase peptide synthesis steps for the production of embodiments of the present compounds, namely 2-lminobiotinyl-L-propargylglycyl-w-lysine (2-IB-alkyne) and 2-Iminobiotinyl-4-azido-L-homoalaninyl-w-lysine (2-IB-azide). Molecular structure of 2-IB-alkyne **(A)** and 2-IB-azide **(B). C.** Synthetic peptides used for the synthesis of 2-IB-alkyne and 2-IB-azide. **D.** Synthesis steps for the production of 2-IB-alkyne and 2-IB-azide. Abbreviations: DCM (Dichloromethane; CAS no. 75-09-2), DIEA (N,N-Diisopropylethylamine; CAS no. 7087-68-5), DMF (N,N-Dimethylformamide; CAS no. 68-12-2), HATU ([O-(7-Azabenzotriazol-1-yl)-N,N,N',N'-tetramethyluronium-hexafluorphosphat]; Cas no. 148893-10-1), TFA (Trifluoroacidic acid; Cas no. 76-05-1) and TIPS (Triisopropylsilanol; Cas no. 17877-23-5).
**Figure 5****:** Fluorescent imaging of cells labelled with the exemplary compounds 2-IB-azide and 2-IB-alkyne filled neurons reveals fine dendritic and axonal structures comparable to biocytin filled cells. **A.** Confocal images of dissociated primary cortico-hippocampal rat neurons filled with Biocytin (2.69 mM), 2-IB-azide (6 mM) and 2-IB-alkyne (6 mM) and visualized by fluorophore coupled reporter molecules (biocytin: avidin conjugated Alexa Fluor 647). Fine dendritic processes (white rectangles) and axon (white arrows) are highlighted. Enlargements of white rectangle regions are shown in **B.** (scale bar = 10 µm). Note, that fine spiny dendritic processes are revealed by 2-IB-azide and 2-IB-alkyne filling. Enlarged axon segments are highlighted in **C.** (scale bar = 10 µm). **D.** Image showing combined Biocytin, 2-IB-azide, 2-IB-alkyne filling of neighbouring neurons in the same dissociated culture. Overlapping processes can be differentiated by the different fluorescent reporters.

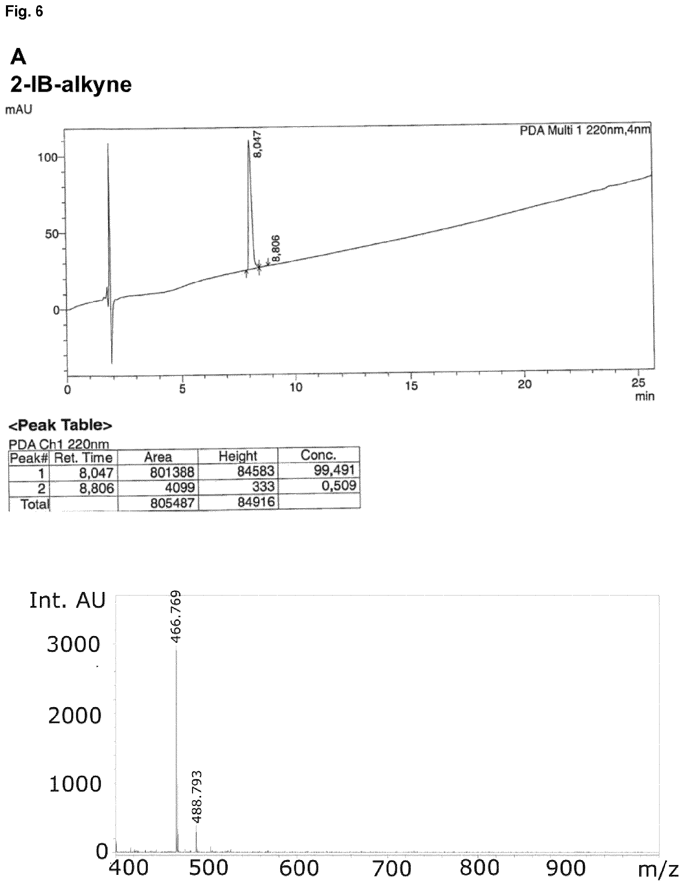

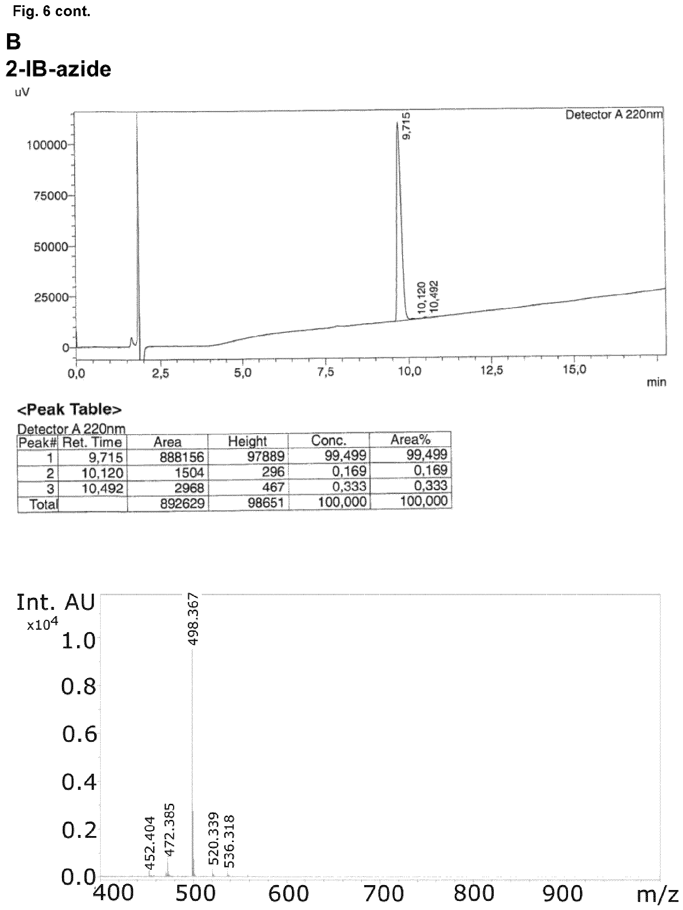

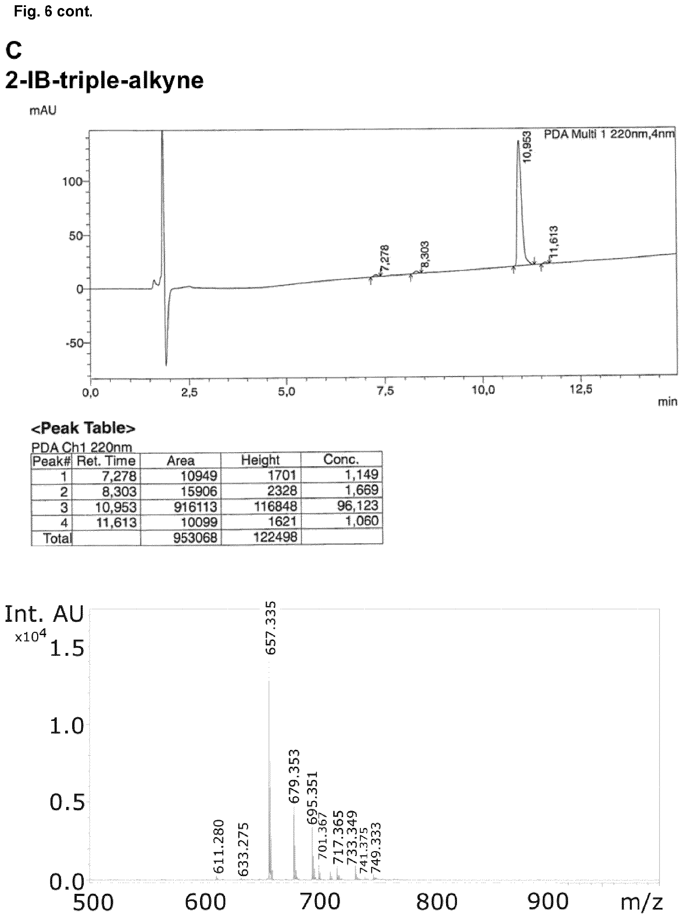

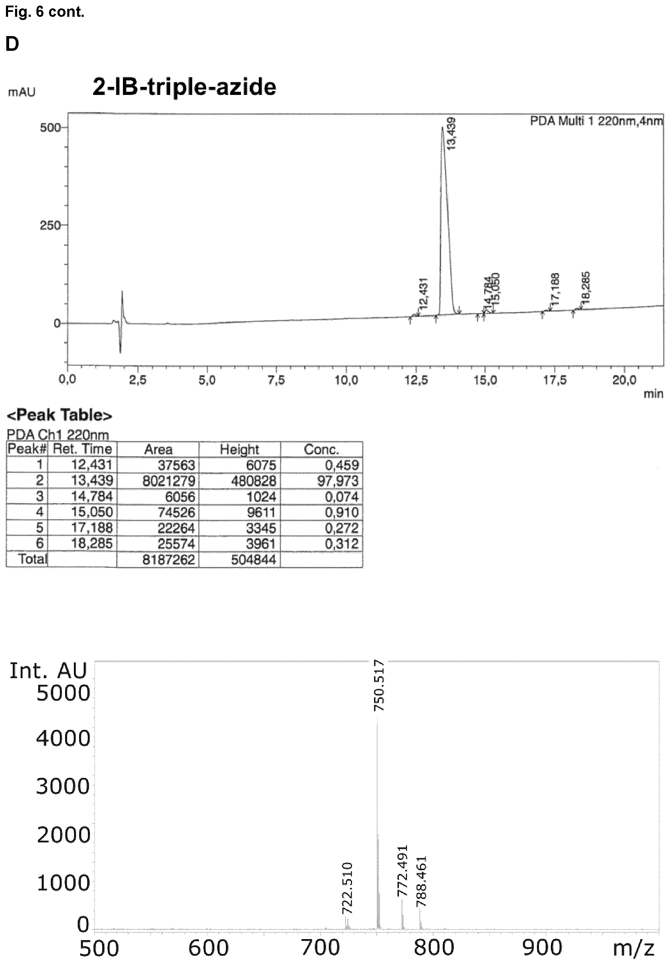

**Figure 6****: A - D** chromatogram (respective graph on top) and HPLC (respective graph on bottom) results demonstrating molecule development and purity, with 2-IB-alkyne, 2-IB-azide, 2-IB-triple-alkyne and 2-IB-triple-azide. HPLC graphs: X-axis m/z, Y-axis: intensity.

### EXAMPLES

The invention is further described by the following examples. These are not intended to limit the scope of the invention but represent preferred embodiments of aspects of the invention provided for greater illustration of the invention described herein.

### Example 1

### Solid-phase peptide synthesis

The stepwise solid-phase synthesis of the peptide acids was performed manually, according to the conventional Fmoc/tBu strategy. The solid support used was 2-Cl-Trityl-Harz (1.4mmol/g) from Rapp Polymere GmbH. The first amino acid was coupled to the resin for 2hours. Dried methylene chloride (DCM) was used as solvent and Diisopropylethylamine (DIEA) as base. The clickable amino acids were coupled twice according to standard methodologies with HATU as a coupling reagent. Fmoc deprotection was carried out with 20% piperidine in DMF. The iminobiotin residue was coupled overnight according to standard methods. The crude peptides were separated from their resins by treatment with 95% TFA, 2.5% water and 2.5% TIPS for 1.5 hours. After evaporation of the solvent, the crude peptide was obtained after addition of diethyl ether.

After Lyophilisation of the peptide, preparative purification by high-pressure liquid chromatography (HPLC) was carried out on a Shimadzu LC-8A system with a Kromasil 100, C18 (245x50mm) column (Buffer A: 0.2% TFA in water, Buffer B: 0.2% TFA in water:acetonitrile, 1:4). The purity was checked with HPLC and mass spec (Figure 6).

### Example 2

### Material and methods

All procedures and animal maintenance and handling were performed in accordance with institutional guidelines, the German Animal Welfare Act and the European Council Directive 86/609/EEC regarding the protection of animals, in the presence of permissions from local authorities (LaGeSo Berlin, T-CH 0040/20).

Primary dissociated cortico-hippocampal neurons were prepared from WT Wistar Rat pups at postnatal day (P) 0 - 2. Tissue was excised and then dissociated using papain (1.5 mg/mL; Merck) for 30 mins at 37 ∘C, before trituration in bovine serum albumin (10 mg/ml; Merck). Cells were then resuspended and cultured in Neurobasal A medium (supplemented with 1 × B27, 1 × Glutamax, and 100 U/ml Penicillin- Streptomycin; Thermo Fisher Scientific). Cells were grown on 12-mm glass coverslips (Roth) previously coated for 1 h with poly-L-lysine hydrobromide (20 µg/ml; Merck) in 24-well cell culture plates (BD Falcon). Cells were grown in humidified conditions at 37 ∘C and 5 % CO2 until experimentation (Methods described in detail Turko et al., 2019).

For patch-clamp experiments, cells were patched in dissociated neuronal cell cultures from day in vitro (DIV) 14 onwards. Cell cultures were submerged and superfused with artificial cerebrospinal fluid (ACSF: 125 mM NaCl, 25 mM NaHCO₃, 2.5 mM KCI, 1.25 mM NaH₂PO₄, 2 mM CaCl₂, 1 mM MgCl₂ and 25 mM D-glucose and 1 mM sodium pyruvate and 1 mM sodium ascorbate; equilibrated with 95% O₂ and 5% CO₂ gas mixture at 30-32 °C).

Recording electrodes were pulled from borosilicate glass with filament (outer diameter, 2 mm; wall thickness, 0.5 mm) with a horizontal pipette puller (P-97, Sutter Instruments, Novato, USA). Pipettes were filled with an internal solution containing: 130 mM potassium gluconate, 0.2 mM EGTA, 10 mM KCI, 10 mM HEPES, 2 mM MgCl₂, 2 mM NA₂ATP, 0.3 mM Na₂GTP, 1 mM Phosphocreatine disodium salt hydrate and 0.1% Biocytin (wt/vol) (Molecular Probes, Eugene, USA); pH was adjusted to 7.3 using potassium hydroxide (KOH).

Filled electrodes had a series resistance between 3 and 5 MΩ. Voltage-clamp and current-clamp experiments were performed using an Axon Digidata 1550 digitizer (Axon Instrument) and Multiclamp 700B amplifier under the control of the Axon MultiClamp Commander software (Molecular Devices, Sunnyvale, USA),

To measure action potential (AP) firing, rheobase (to measure rheobase, voltage responses were recorded by the injection of current pulse starting at 0 pA at a +20 pA step size) and input resistance, voltage responses were recorded following the injection of hyperpolarizing and depolarizing current pulses (500 ms, -250 - +250 pA and step size: +50 pA). Pipette capacitance and series resistance compensation (bridge balance) were applied during current-clamp recordings. Resting membrane potential was determined in I=0 mode.

To fill cells with 2-IB-alkyne, 2-IB-azide or biocytin, compound powders were dissolved in intracellular solutions prior to patching, at 6 mM, 6 mM, and 2.69 mM respectively. Cells were allowed to fill for at least 15 minutes before an "outside out patch" was created to seal the membrane. Following this, cells were transferred to 4% paraformaldehyde (PFA) in phosphate-buffered saline (PBS) solution for 15 minutes. Cells were then washed for 10 minutes in 0.1 M phosphate buffer, before 3 × 10-minute washes in PBS solution. Cells were then incubated overnight at 4°C in 0.1-0.2% Triton X-100 (in PBS) with streptavidin-conjugated Alexa Flour 488. Following 3 further 10-minute washes in PBS, cells were then incubated in "click-solution" containing 0.2 mM THPTA, 20mM Sodium ascorbate, 0.2 mM Copper (II) sulphate, 1 µM) Sulfo-Cyanine5 azide, and/ or 1 µM) Sulfo-Cyanine3 alkyne in PBS at RT for 10 minutes. After 3 × 10-minute PBS washes, coverslips were mounted in fluoromount-G medium (Invitrogen, USA). Labelled cells were then examined using a confocal microscope (FV1000, Olympus, Hamburg, Germany). The result of imaging is shown in Fig. 1C, 2B and C (image from mouse hippocampus) and Fig. 5.

### Results

In a representative example of labelling neurons using the present tracer compounds neurons were filled with the exemplary tracer compounds 2-IB-azide and 2-IB-alkyne to detect fine dendritic and axonal structures in comparison to biocytin filled cells.

First, dissociated primary cortico-hippocampal rat neurons filled with Biocytin (2.69 mM), 2-IB-azide (6 mM) and 2-IB-alkyne (6 mM) and visualized by fluorophore coupled reporter molecules (biocytin: avidin conjugated Alexa Fluor 647). The confocal images shown in Figure 5A illustrate that the present tracer compounds enable the detection of fine dendritic processes (white rectangles) and axon (white arrows). Enlargements of white rectangle regions are shown in Figure 5B (scale bar = 10 µm). Note, that fine spine-covered dendritic processes are revealed by 2-IB-azide and 2-IB-alkyne filling.

In additions, axon segments could be detected and are highlighted in Figure 5C (scale bar = 10 µm).

Subsequently, neighbouring neurons in the same dissociated culture were stained (filled) with either Biocytin, 2-IB-azide or 2-IB-alkyne. The image shown in Figure 5D illustrates that overlapping processes can be differentiated by the different fluorescent reporters.

In addition, electrophysiological recordings were performed showing no significant alterations of the intrinsic electrical properties of neurons filled with Biocytin, 2-IB-azide and 2-IB-alkyne. The voltage responses of filled neurons to hyperpolarizing and depolarizing current pulses (duration: 500 ms. From -250 pA to 0 pA, 50 pA steps. Suprathreshold pulse: 250 pA) are shown in Figure 3A.

In total, 31 neurons were investigated with biocytin (n = 31), 33 neurons with 2-IB-azide (n = 33) and 34 neurons with 2-IB-alkyne (n = 34). Figure 3A further shows violin plots of the resting membrane potential (RMP), input resistance (Rin), rheobase, AP peak from threshold and AP half-height width measurements made from filled neurons. Importantly, there were no significant differences observed between biocytin filled cells and either 2-IB-azide or 2-IB-alkyne filled cells. For calculating the violin plots Kruskal-Wallis non-parametric comparison with Dunn's multiple comparison testing was performed.

### REFERENCES

Athappilly FK, Hendrickson WA. Crystallographic analysis of the pH-dependent binding of iminobiotin by streptavidin. Protein Sci. 1997 Jun;6(6):1338-42. doi: 10.1002/pro.5560060623. PMID: 9194195; PMCID: PMC2143720.
Booker SA, Song J, Vida I. Whole-cell patch-clamp recordings from morphologically- and neurochemically-identified hippocampal interneurons. J Vis Exp. 2014 Sep 30;(91):e51706. doi: 10.3791/51706. PMID: 25350149; PMCID: PMC4672954.
Hamill OP, Marty A, Neher E, Sakmann B, Sigworth FJ. Improved patch-clamp techniques for high-resolution current recording from cells and cell-free membrane patches. Pflugers Arch. 1981 Aug;391(2):85-100. doi: 10.1007/BF00656997. PMID: 6270629.
Hanani M. Lucifer yellow - an angel rather than the devil. J Cell Mol Med. 2012 Jan;16(1):22-31. doi: 10.1111/j.1582-4934.2011.01378.x. PMID: 21740513; PMCID: PMC3823090.
Ling C, Hendrickson ML, Kalil RE. Resolving the detailed structure of cortical and thalamic neurons in the adult rat brain with refined biotinylated dextran amine labeling. PLoS One. 2012;7(11):e45886. doi: 10.1371/journal.pone.0045886. Epub 2012 Nov 5. PMID: 23144777; PMCID: PMC3489877.
Orr GA. The use of the 2-iminobiotin-avidin interaction for the selective retrieval of labeled plasma membrane components. J Biol Chem. 1981 Jan 25;256(2):761-6. PMID: 6161128.
Parekh R, Ascoli GA. Neuronal morphology goes digital: a research hub for cellular and system neuroscience. Neuron. 2013 Mar 20;77(6):1017-38. doi: 10.1016/j.neuron.2013.03.008. Erratum in: Neuron. 2013Apr 10;78(1):206. PMID: 23522039; PMCID: PMC3653619.
Peng Y, Barreda Tomás FJ, Klisch C, Vida I, Geiger JRP. Layer-Specific Organization of Local Excitatory and Inhibitory Synaptic Connectivity in the Rat Presubiculum. Cereb Cortex. 2017 Apr 1;27(4):2435-2452. doi: 10.1093/cercor/bhx049. PMID: 28334142; PMCID: PMC5390487.
Peng Y, Barreda Tomas FJ, Pfeiffer P, Drangmeister M, Schreiber S, Vida I, Geiger JRP. Spatially structured inhibition defined by polarized parvalbumin interneuron axons promotes head direction tuning. Sci Adv. 2021 Jun 16;7(25):eabg4693. doi: 10.1126/sciadv.abg4693. PMID: 34134979; PMCID: PMC8208710.
Steiner LA, Barreda Tomás FJ, Planert H, Alle H, Vida I, Geiger JRP. Connectivity and Dynamics Underlying Synaptic Control of the Subthalamic Nucleus. J Neurosci. 2019 Mar 27;39(13):2470-2481. doi: 10.1523/JNEUROSCI.1642-18.2019. Epub 2019 Jan 30. PMID: 30700533; PMCID: PMC6435833.
Takeuchi K, Yoshii K. Effect of superoxide derived from lucifer yellow CH on voltage-gated currents of mouse taste bud cells. Chem Senses. 2008 Jun;33(5):425-32. doi: 10.1093/chemse/bjn009. Epub 2008 Mar 3. PMID: 18319263.
Tayri-Wilk T, Slavin M, Zamel J, Blass A, Cohen S, MotzikA, Sun X, Shalev DE, Ram O, Kalisman N. Mass spectrometry reveals the chemistry of formaldehyde cross-linking in structured proteins. Nat Commun. 2020 Jun 19;11(1):3128. doi: 10.1038/s41467-020-16935-w. PMID: 32561732; PMCID: PMC7305180.
Tasker JG, Hoffman NW, Dudek FE. Comparison of three intracellular markers for combined electrophysiological, morphological and immunohistochemical analyses. J Neurosci Methods. 1991 Jul;38(2-3):129-43. doi: 10.1016/0165-0270(91)90163-t. PMID: 1723776.
Thomson AM, Armstrong WE. Biocytin-labelling and its impact on late 20th century studies of cortical circuitry. Brain Res Rev. 2011 Jan 7;66(1-2):43-53. doi: 10.1016/j.brainresrev.2010.04.004. Epub2010Apr24. PMID: 20399808; PMCID: PMC2949688.Turko P, Groberman K, Browa F, Cobb S, Vida I. Differential Dependence of GABAergic and Glutamatergic Neurons on Glia for the Establishment of Synaptic Transmission. Cereb Cortex. 2019 Mar 1;29(3):1230-1243. doi: 10.1093/cercor/bhy029. PMID: 29425353.
Wang Q, Chan TR, Hilgraf R, Fokin VV, Sharpless KB, Finn MG. Bioconjugation by copper(I)-catalyzed azide-alkyne [3 + 2] cycloaddition. JAm Chem Soc. 2003 Mar 19;125(11):3192-3. doi: 10.1021/ja021381e. PMID: 12630856.

## Claims

1. A tracer compound for whole-cell labelling, comprising:
a. a non-streptavidin-binding biotin analogue,
b. at least one connecting moiety comprising at least one functional group configured for a click-chemistry reaction, and
c. at least one cross-linkable moiety.

2. The tracer compound according to claim 1, wherein the cross-linkable moiety is a (para)formaldehyde cross-linkable group, comprising an amine group, preferably an amino acid, such as lysine.

3. The tracer compound according to any one of the preceding claims, wherein the non-streptavidin-binding biotin analogue enables the tracer compound to diffuse within a biological cell with essentially the same properties as biocytin and/or neurobiotin.

4. The tracer compound according to any one of the preceding claims, wherein the non-streptavidin-binding biotin analogue enables the tracer compound to diffuse within a biological cell, enabling the detection of cellular protrusions, such as dendrites, dendritic spines and axons, using microscopic imaging.

5. The tracer compound according to any one of the preceding claims, wherein the non-streptavidin-binding biotin analogue is 2-iminobiotin (2-IB).

6. The tracer compound according to any one of the preceding claims, wherein the at least one functional group configured for a click-chemistry reaction is selected from the group comprising azides, alkynes, strained alkynes, tetra azides, trans-cycloctenes, tetrazines and alkenes, strained alkenes, tetra azides, cyclooctynes, phosphines, ketones, aldehydes, amines and tetrazines, and preferably enables the generation of a triazole conjugation.

7. The tracer compound according to any one of the preceding claims, wherein the compound comprises more than one connecting moiety.

8. The tracer compound according to any one of the preceding claims, wherein the compound is 2-lminobiotinyl-L-propargylglycyl-w-lysine (2-IB-alkyne) or 2-lminobiotinyl-4-azido-L-homoalaninyl-w-lysine (2-IB-azide).

9. The tracer compound according to any one of the preceding claims, wherein the compound comprises a label, preferably a fluorescent dye, coupled to the connecting moiety, preferably via a triazole conjugation resulting from a click-chemistry reaction between an alkyne and an azide.

10. The tracer compound according to any one of the preceding claims, wherein the compound does not impair the electrophysiological properties of the cell (is electrophysiologically-neutral).

11. A method for constructing a tracer compound according to any one of the preceding claims, comprising the coupling of:
a. a non-streptavidin-binding biotin analogue, preferably 2-iminobiotin,
b. at least one connecting moiety comprising at least one functional group configured for a click-chemistry reaction, preferably one or more of an i) azide and/or ii) alkyne moiety, and
c. a cross-linkable moiety, preferably a (para)formaldehyde cross-linkable moiety comprising an amine group,
i. to form a tracer compound for whole-cell labelling,
d. and optionally coupling a label, preferably a fluorescent dye, to said functional group, wherein the dye is preferably coupled via a click-chemistry reaction, to form an electrophysiological neutral dye.

12. A method for labelling a cell, comprising:
a. injecting a tracer compound according to any one of claims 1-10 into the cell, preferably by applying a current, or applying the compound juxtacellularly (externally to the cell),
b. optionally, during which, determining electrophysiological properties (performing electrophysiology measurements) of the cell,
c. allowing the tracer compound to diffuse through and/or around the cell,
d. fixing the cell and tracer compound with a crosslinker, preferably a formaldehyde, more preferably paraformaldehyde,
e. optionally permeabilizing the cell,
f. adding a label into the cell, or juxtacellularly, that reacts with and becomes coupled to the functional group of the connecting moiety of the tracer compound, and
g. detecting and imaging the label and/or the cell.

13. The method according to any one of claim 12, wherein the cell is an electrically-active cell, preferably a neuron, a glial cell, or muscle cell, preferably a cell of the CNS and PNS, or any other cell type possessing ion channels, an electrochemical potential, or electrical activity.

14. The method according to any one of claims 12-13, wherein biocytin and/or neurobiotin are additionally injected into the cell or nearby cell, or applied juxtacellularly, and an additional label coupled to streptavidin is added to the cell, and subsequently detected after binding to biocytin and/or neurobiotin.

15. A kit for carrying out the method according to any one of claims 12-14, comprising:
a. at least one tracer compound according to any one of claims 1-10, and
b. one or more labels (preferably comprising a fluorescent dye) comprising at least one functional group configured for a click-chemistry reaction,
c. and optionally one or more of biocytin, neurobiotin, a label (preferably comprising a fluorescent dye) coupled to streptavidin, a crosslinker (preferably (para)formaldehyde), a cell-permeabilizing agent (such as a detergent), suitable buffers, and reagents for performing a click-chemistry reaction, preferably copper(II) sulfate, sodium ascorbate and Tris(benzyltriazolylmethyl)amine (THPTA).
